# EUROPEAN PATENT SPECIFICATION

(11) **EP 1 590 016 B1**
(45) Date of publication and mention of the grant of the patent: **17.10.2012**
(21) Application number: 04703227.1
(22) Date of filing: 19.01.2004
(51) Int. Cl.: A61M 1/16, A61M 1/36

(54) **INTEGRATED BLOOD TREATMENT MODULE AND EXTRACORPOREAL BLOOD TREATMENT APPARATUS.**
INTEGRIERTES BLUTBEHANDLUNGSMODUL UND EXTRAKORPORALES BLUTBEHANDLUNGSGERÄT
MODULE DE TRAITEMENT SANGUIN INTEGRE ET APPAREIL DE TRAITEMENT SANGUIN EXTRACORPOREL.

(30) Priority: 07.02.2003 IT MI20030211
(43) Date of publication of application: 02.11.2005
(73) Proprietor: Gambro Lundia AB, 22 643 Lund (SE)
(72) Inventor: CHEVALLET, Jacques, F-69360 Sérezin Du Rhone (FR); DUCHAMP, Jacques, F-69500 Bron (FR); ABERKANE, Aziz, F-69150 Decines (FR); MEYSSONNIER, Gabriel, F-38460 Dizimieu (FR); DELNEVO, Annalisa, I-40019 Sant'Agata Bolognese (IT); TONELLI, Claudio, I-41100 Modena (IT); ZACCARELLI, Massimo, I-41038 San Felice Sul Panaro (IT); RIBOLZI, Francesco, I-21100 Varese (IT); BARALDI, Vincenzo, I-46026 Quistello (IT); POUCHOULIN, Dominique, F-01390 Tramoyes (FR)
(74) Representative: Ponzellini, Gianmarco
(86) International application number: PCT/IB2004/000104
(87) International publication number: WO 2004/069308

(56) References cited:
- EP-A- 0 611 227
- WO-A-99/13926
- US-A- 4 436 620

## Description

### Background of the Invention.

The present invention relates to an integrated blood treatment module and to an extracorporeal blood treatment apparatus that can receive said integrated module.

As is known, in order to carry out extracorporeal blood treatments such as for instance haemodialysis, haemofiltration, haemodiafiltration, plasmapheresis, extracorporeal blood oxygenation, extracorporeal blood filtration or other treatments, it is necessary to provide for at least an extracorporeal circuit through which the blood flows and is conveyed towards a treatment unit; the treated blood is then carried back to the patient's cardiovascular system. Referring by way of example to a dialysis treatment, the extracorporeal circuit used comprises: a dialysis filter consisting of a housing body defining at least a first and a second chamber separated from each other by a semipermeable membrane, a blood intake line leading to the first chamber of the dialysis filter and a blood return line designed to receive blood coming out of the first chamber and to carry it back to the patient. The second chamber of the dialysis filter is then connected to a circuit for the circulation of a dialysis liquid designed to receive the impurities that are present in the blood and the excess fluid that has to be removed from the patient's blood.

Currently, in apparatus for extracorporeal blood treatments all the lines designed for the circulation of the dialysis liquid are housed within the dialysis apparatus, whereas the lines constituting the extracorporeal blood circuit are replaced at every treatment and suitably connected to the dialyzing filter, which can be replaced either at every treatment or from time to time, as required.

From a structural point of view the dialysis filter, the lines for the circulation of the dialyzing liquid and the lines constituting the intake branch carrying the blood back to the patient consist of separate parts that are connected and cooperate during operation after being suitably assembled.

There are also apparatus that are available on the market at present, designed in particular for intensive treatment of kidney failure, which are advantageously equipped with integrated modules comprising a support structure, a dialyzing filter engaged to the support structure by means of a suitable support projecting from said structure, as well as a hydraulic circuit comprising the tubes that are necessary to define the blood suction and return lines leading to the patient, the possible lines for the infusion of anticoagulant or substitution liquids, the intake line for the dialysis liquid and the discharge line for the liquid coming out of the second chamber of the dialyser.

The integrated modules described above enable an easy and immediate association of the lines to the treatment apparatus and do not require any connection between the treatment unit, such as for instance a dialysis filter, and the various tubes or lines designed to convey blood and other fluids. Moreover, said integrated modules enable the removal both of the tubes conveying the blood and of the tubes conveying other fluids at the end of the treatment. In other words, thanks to a simple loading and connecting operation of the terminals and of the fluid conveyance lines to the corresponding sources such as bags or others, the user can install a dialysis apparatus. Analogously, once the treatment step is over, by simply disconnecting and disassembling the integrated module from the blood treatment apparatus in few operations, the operator can completely eliminate both the extracorporeal circuit and the blood treatment unit, as well as the tubes for the circulation of possible infusion liquids and of the dialysis liquid. The easy installation of said modules ensures an efficiency and a speed that are certainly advantageous for intensive treatments where the personnel, who might not be conversant with the use of blood treatment apparatuss, can thus operate rapidly and with a high reliability.

In particular, it is known about integrated modules for extracorporeal blood treatment in which a quadrangular plate, thanks to the use of an auxiliary engagement structure, centrally carries the blood treatment filter and also supports on each of its sides four tube lengths of corresponding lines of the fluid distribution circuitry.

In particular, each of the four sides has two connectors to which a respective tube length, basically semicircular, is secured; each length can be engaged by a respective peristaltic pump.

The four ring-shaped tube lengths extend away from the four sides and all have the same shape and size.

In particular, the part of the module consisting of the support plate and of the U-shaped tube lengths is symmetrical with respect to two orthogonal axes.

The arrangement referred to above, though being widely used today in integrated modules designed for intensive therapy apparatus, has proved to be susceptible of several improvements.

First of all, it should be noted that the particular relative arrangement of the various U-shaped tube lengths and, therefore, of the respective pumps supported by the apparatus do not allow to optimize the lengths of the various portions of tubes in which blood, dialysis fluids, waste fluids, etc.

Furthermore, it is not possible to use pumps with larger size (which would thus involve U-shaped tube lengths with larger size) in any of the lines without prejudicing the compactness and the overall dimensions of the integrated module.

Eventually, it should be noted that the module at the state of the art is necessarily designed for a maximum of four peristaltic pumps for conveying the respective fluids, since other infusion lines beyond those that are already provided cannot be installed.

EP A 0 611 227 discloses an integrated fluid treatment module comprising a support element, a fluid distribution circuit associated to the support element and comprising a blood line and further fluid lines. A portion of the blood line is fastened to a first zone of the support element and defines a U-shaped tube length designed to cooperate with a respective blood pump. The further fluid lines are fastened to a second zone of the support element and each of them defines a U-shaped tube length designed to cooperate with a respective pump.

WO 99/13926 discloses a cassette assembly with a number of outwardly extending tubes that interconnect various integral fluid conduits and tubing assemblies. The outwardly extending tubes may be interconnected wherein each loop is engaged with a pumping device. One of the outwardly extending tubes is longer than the other ones.

US 4,436,620 discloses a one-piece hydraulic circuit for use with a blood dialyzer comprising a rigid, unitary member defining spaced first, second and third chambers therein. A first port communicates with the first chamber, and is adapted for connection with a venous line of a patient. A second port also communicates with the first chamber, and is adapted for connection with the outlet of a blood dialyzer. The second chamber communicates with a third port which in turn is adapted for connection with an arterial line of the patient. The second chamber also communicates with a fourth port adapted for connection with an end of blood pump tubing.

### Summary of the Invention.

The present invention therefore aims at solving basically the drawbacks and operating limitations referred to above.

A first aim of the invention is to carry out an integrated module in which the arrangement of the various tube lengths allows a high compactness of said module as well as an optimal distribution of the lengths of the various lines of the hydraulic circuit.

A further aim of the invention is to enable the presence of at least a blood line in which the biological fluid can be conveyed by pumps with larger radial size without damaging the compactness of said integrated module.

Finally, an auxiliary aim of the invention is to carry out an integrated module that can protect the various U-shaped tube lengths on which the peristaltic pumps act, thus protecting also the latter while the apparatus is working.

These and other aims, which shall be evident in the course of the present description, are basically achieved by an integrated module and by an apparatus as described in the appended claims.

Further characteristics and advantages will be clearer from the detailed description of a preferred though not exclusive embodiment of a support element, of an integrated module and of a corresponding apparatus for extracorporeal blood treatment according to the present invention.

### Brief Description of the Drawings.

This description will be given below with reference to the appended drawings, which are provided as a mere guidance and are therefore not limiting, in which:
- Figure 1 shows a schematic view of a hydraulic circuit carried out by an apparatus and a module according to the present invention;
- Figure 2 shows a view from above of a support element according to the present invention;
- Figure 3 shows a section of the module of Figure 2 according to line III-III;
- Figure 4 shows a further section of the element of Figure 2 according to line IV-IV;
- Figure 5 shows again a section according to line V-V of Figure 2;
- Figure 6 shows a section according to line VI-VI of the support element of Figure 2;
- Figure 7 shows a perspective view from a first side of the support element of Figure 2;
- Figure 7a shows an enlarged detail of the element of Figure 7;
- Figure 8 shows a perspective view from the opposite side of the support element of Figure 7;
- Figure 8a shows an enlarged detail of the element of Figure 8;
- Figure 9 shows a perspective view of an integrated module according to the present invention;
- Figure 9a shows an enlarged detail of the module of Figure 9;
- Figure 10 shows a perspective view from the opposite side of the module of Figure 9;
- Figure 10a shows an enlarged detail of the module of Figure 10;
- Figure 11 shows the integrated module of Figure 10 to which a blood treatment unit can be associated;
- Figure 12 shows a section of a connector of the support element and of a counter-connector of the blood treatment unit;
- Figure 13 shows a further section of a connector according to the present invention;
- Figure 14 shows a schematic view of the integrated module that can be associated to the apparatus, equipped with the hydraulic circuitry;
- Figure 15 shows an apparatus according to the present invention to which an integrated module can be associated; and
- Figure 16 shows a front view of the apparatus of Figure 15 with an integrated module without the hydraulic circuitry thereto associated.

### Detailed Description.

With reference to the figures mentioned above the numeral 4 globally refers to a support element according to the present invention.

Conversely, the numeral 1 refers to an integrated module (combination of a support element 4, a distribution circuitry 15 and a blood treatment unit 5) that can be used together with extracorporeal blood treatment apparatus 2 according to the present invention.

As can be inferred from the appended Table 1, the global hydraulic circuit carried out thanks to the cooperation between the integrated module and the apparatus consists of a blood line or circuit 44, which takes blood from a patient, for instance by means of a catheter introduced into a vein or artery of said patient, and through at least an intake branch or inlet line 46 carries said blood, for instance continuously, to a filtration unit 5.

Then the blood passes through a primary chamber of said filtration unit 5 and through an outlet line 47 the treated blood is carried back to the patient.

The connection with an auxiliary pre-infusion line 50 is provided immediately downstream from the blood collecting zone on the inlet line 46.

In particular, the apparatus is equipped with at least a secondary fluid container or bag 87 for supplying the pre-infusion line 50; by using corresponding means for conveying fluid, in the example shown comprising an auxiliary pre-infusion pump 3e, for instance a peristaltic pump, it is possible to control the fluid flow within said line by introducing said fluid directly into the blood by means of a direct connection to the inlet line 46.

Generally, the secondary fluid container 87 can house a suitable biological fluid for a pre-infusion, however said bag 87 can also contain an anticoagulant, generally having such a topical nature as to ensure a particular working of the apparatus as shall be explained below in further detail.

After defining a direction of blood circulation 88 from the inlet line 46 towards the filtration unit and from the latter through the outlet line 47 towards the patient, a known blood pressure sensor 89, which shall not be described in further detail, is placed immediately downstream from the auxiliary pre-infusion line 50.

The blood circuit 44 therefore comprises means for conveying fluid, i.e. in this particular case at least a blood pump 3 a for controlling and managing the suitable blood flow in the circuit.

Also the blood pump 3a is generally a peristaltic pump.

Following the direction of blood circulation 88, there is then a device 90 for administering an anticoagulant, for instance a syringe containing suitable doses of heparin.

The blood then passes through another pressure sensor 91 controlling the correct flow within the blood circuit.

After passing through a main chamber of the filtration unit 5, where the suitable exchanges of substances, molecules and fluids occur by means of a semipermeable membrane, the treated blood enters the outlet line 47 first passing through a gas separating device (generally air) 52 commonly known as "bubble trap", designed so as to ensure the removal of substances or air bubbles present in the blood or introduced into the blood during treatment.

The treated blood getting out of the separating device 52 then passes through an air bubble sensor 92 verifying the absence of said dangerous formations within the treated blood that has to be re-introduced in the patient's blood circulation.

Immediately downstream from the bubble sensor 92 there is an element 93 which, in case of alarm, can block the blood flow towards the patient.

In particular, should the bubble sensor 92 detect the presence of anomalies in the blood flow, the apparatus through the element 93 (be it a tap, a clamp or similar) would be able to block immediately the passage of blood so as to avoid any consequence to the patient.

Downstream from said element 93 the treated blood is then carried back to the patient undergoing therapy.

The extracorporeal blood treatment apparatus shown above is then equipped with a fluid circuit 94, which is also provided with at least an inlet line 48 leading into the filtration unit 5 and with an outlet line 45b from the filtration unit.

At least a primary fluid container 95 is designed to supply the inlet line 48 of the fluid circuit 94 (generally the primary fluid container 95 shall consist of a bag containing a suitable dialyzing liquid).

The inlet line 48 then comprises means for conveying fluid such as a least a pump 3c (in the embodiment shown a peristaltic pump) for controlling the flow of liquid from the bag 95 and for defining a direction of circulation 96.

Downstream from the pump 3c in the direction of circulation 96 there is a branching 85 splitting the fluid circuit 94 up into an intake branch 76 and into an infusion branch 77.

In particular, the infusion branch 77 is connected to the outlet line 47 of the blood circuit 44.

In other words, by means of said infusion branch 77 it is possible to obtain a post-infusion directly in the blood line using the content of the primary fluid container 95.

Conversely, the intake branch 76 conveys the fluid directly to the filtration unit and in particular to a secondary chamber of said unit.

The fluid circuit 94 is further equipped with selecting means 97 for determining the percentages of fluid flow within the infusion branch 77 and the intake branch 76.

Generally said selecting means 97, usually placed near the branching 85, can be positioned at least between a first operating condition in which they allow the passage of fluid in the intake branch 76 and block the passage in the infusion branch 77, and a second operating condition in which they allow the passage of fluid in the infusion branch 77 and block the passage in the intake branch 76.

In other words, said selecting means 97 can consist of a valve element operating on the fluid circuit 94 by alternatively blocking the passage of fluid in either branch.

It is also evident that it might be provided for suitable selectors, which are able to establish a priori the amount of liquid that has to pass through both branches simultaneously.

It will also be possible to vary the percentages of fluid in either branch as a function of time and of the pre-established therapies.

The dialyzing liquid through the intake branch 76 gets into a secondary chamber of the filtration unit 5.

In particular, the primary chamber through which the blood flow passes is separated from the secondary chamber through which the dialyzing liquid passes by means of a semipermeable membrane ensuring the suitable passage of the dangerous substances/molecules and of fluid from the blood towards the dialyzing liquid mainly by means of convection and diffusion processes, and also ensuring through the same principles the passage of substances/molecules from the dialyzing liquid towards the blood.

The dialysis fluid then gets into the outlet line 45 and passes through a suitable pressure sensor 98 whose function is to control the working of said line.

Then there are means for conveying fluid, for instance a suction pump 3b controlling the flow in the outlet line 45 within the fluid circuit 94.

Also said pump will generally be a peristaltic pump.

The fluid to be eliminated then passes through a blood detector and is conveyed into a collection container or bag 99.

Further analyzing the peculiar circuit of the apparatus according to the invention, note the presence of at least another infusion line 51 acting on the outlet line 47 of the blood circuit 44.

In particular, the infusion fluid is taken from at least an auxiliary container 200 and is sent directly to the outlet line 47 of the blood circuit 44 through means for conveying fluid, generally an infusion pump 3d controlling its flow (in the example a peristaltic pump).

In particular and as can be observed in the appended figure, the infusion liquid can be introduced directly into the gas separating device 52.

As can also be inferred, the infusion branch 77 of the fluid circuit 94 and the infusion line 51 are equipped with a common end length 201 letting into the blood circuit 44.

Said intake end length 201 is placed downstream from the infusion pump 3d with respect to a direction of infusion 202 and carries the fluid directly into the bubble trap device 52.

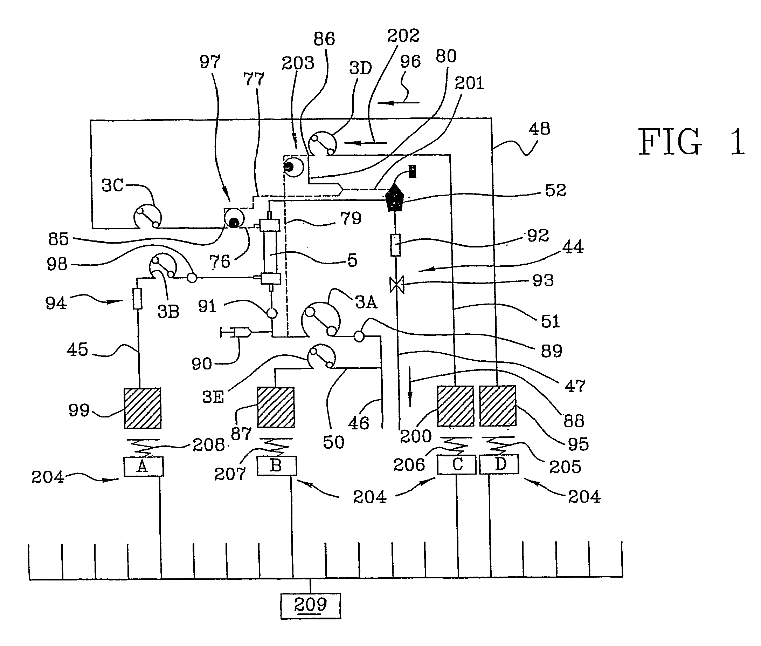

Further referring to the diagram in Figure 1, note the presence within the infusion line 51 of at least a pre-infusion branch 79 connected to the inlet line 46 of the blood circuit 44.

In further detail, downstream from the infusion pump 3d with respect to the direction of infusion 202, there is a branching 86 splitting the infusion line 51 up into pre-infusion branch 79 and post-infusion branch 80.

The pre-infusion branch 79, in particular, carries the fluid taken from the bag 200 on the inlet line 46 of the blood circuit downstream from the blood pump 3a with respect to the direction of circulation 88.

Conversely, the post-infusion branch 80 is connected directly to the common end length 201.

The infusion line 51 further comprises selecting means 203 for determining the percentage of liquid flow to be sent to the post-infusion branch 80 and to the pre-infusion branch 79.

The selecting means 203 placed near the branching 86 can be positioned between at least a first operating condition in which they allow the passage of fluid in the pre-infusion branch 79 and block the passage in the post-infusion branch 80, and at least a second operating condition in which they allow the passage of fluid in the post-infusion branch 80 and block the passage in the pre-infusion branch 79.

Obviously, as in the case of the selecting means 97 present on the fluid circuit 94, also the other selecting means 203 will be able to determine the percentage of fluid that has to pass in each of the two branches and to possibly vary it in time in accordance with the planned therapies. Moreover, the selecting means 97 and the other selecting means 203 will generally though not necessarily be of the same nature.

The apparatus is then equipped with means 204 for determining at least the weight of the primary fluid container 95 and/or of the auxiliary fluid container 200 and/or of the secondary fluid container 87 and/or of the collection container 99.

In particular, said means 204 comprise weight sensors, for instance respective scales 205, 206, 207, 208 (at least an independent one for each fluid bag associated to the apparatus).

In particular, there will be at least 4 of said scales, each pair being independent from the other and measuring the respective weight of a bag.

It should then be pointed out that there is a processing unit or CPU 209 acting on the blood circuit 44 and in particular on the pressure sensor 89, on the blood pump 3a, on the device 90 for heparin infusion, on the other pressure sensor 91, and on the device for detecting the presence of air bubbles 92 and on its respective closing element 93.

Said CPU 209 has also to control the fluid circuit 94 and, in particular, shall be input with the data detected by the scales 205 and concerning the weight of the bag 95 and shall act on the pump 3c, on the selecting means 97, on the pressure sensor 98, then on the suction pump 3b and shall eventually receive the data detected by the scales 208 whose function is to determine the weight of the collection container 99.

The CPU 209 shall also act on the infusion line 51 checking the weight of the auxiliary container 200 (checked by the scales 206) and will be able to control both the infusion pump 3d and the other selecting means 203.

Eventually, the CPU 209 shall also act on the auxiliary pre-infusion line 50 detecting the weight of the secondary fluid container 87 by means of the scales 207 and suitably controlling the pump 3e according to the treatments to be carried out.

Reminding that the above description has been made with the sole purpose of describing the whole of the hydraulic circuit of the extracorporeal blood treatment apparatus, here is a short description of the working of the device.

Once the whole hydraulic circuit and the filtering unit 5 have been correctly associated to the apparatus so that the various peristaltic pumps engage the respective lengths of tubes and that all the sensors have been suitably positioned, and the various bags containing the various fluids have been associated to the corresponding liquid intake/suction lines, and the blood circuit has been connected to a patient's artery/vein, the initial circulation of blood within its circuit is enabled.

Therefore, according to the kind of therapy that has been set, the extracorporeal blood treatment apparatus is automatically started and controlled by the processing unit 209.

If the patient undergoes an ultrafiltration treatment, beyond the blood circuit the suction pump 3b connected to the outlet line of the fluid circuit 94 is started, so as to take by convection a fluid excess in the patient (beyond the dangerous substances/molecules).

Conversely, if the therapy that has been set comprises a haemofiltration treatment, beyond the blood circuit and the suction pump 3b for taking fluids by convection also the pump 3c on the inlet line of the fluid circuit 94 is started and the selecting means 97 placed so as to enable a post-infusion.

Also the infusion line 51 shall be used so as to enable a further addition of liquids to the post-infusion or to enable a suitable pre-infusion.

Conversely, if the treatment involves haemodialysis, the pumps 3c and 3b of the fluid circuit 94 shall be started and the selecting means 97 shall be positioned so as to ensure the passage of the dialyzing liquid only towards the filtration unit 5 so as to take substances and/or molecules and/or liquids by diffusion and possibly by convection if the transmembrane pressure through the filtration unit is other than zero.

Eventually, if a haemodiafiltration treatment has to be carried out, beyond the blood circuit the fluid circuit and therefore the pumps 3c and 3b shall be started, so as to ensure a circulation of the liquid within the filtration unit 5 and also the pump 3d of the infusion line 51 shall be started so as to ensure a pre- or post-infusion.

Obviously, it will be possible to set up different therapies comprising one or more of the treatments referred to above.

In all the treatments described above, possibly except the ultrafiltration treatment, it will be possible to use the auxiliary pre-infusion line for introducing an anticoagulant and/or a suitable infusion liquid into the blood.

Obviously, the anticoagulant can also be administered by means of the suitable device 90 designed for the introduction of heparin into blood.

Concerning this it should be pointed out that the apparatus according to the invention is designed to receive various kinds of syringes according to the amount of anticoagulant to be administered.

Obviously, it is the control unit 209 that, being connected to the various devices, sensors, pumps and being input with the weight data from the various scales, is able - once it is set - to control and automate the whole working of the apparatus.

In further detail, it is possible to set the flows of the various pumps present on the apparatus in accordance with the therapy or therapies to be started.

Obviously, the suitable setting of said flows results in an amount of fluid taken from the patient (weight loss), which will generally be given by the difference between the weight of the liquid that has been collected in the bag 99 and of the liquid circulated in the circuit through the primary fluid container 95, the auxiliary fluid container 200 and the secondary fluid container 87.

In particular, in accordance with the data received by the control unit coming from the various scales (and the theoretical flow rates fixed on each pump of therapy/treatment carried out) the control unit 209 shall control the means for circulating fluid in the various lines by suitably varying the thrust exerted by the various pumps 3a, 3b, 3c, 3d, 3e.

In particular, the signals coming from the scales referred to above 205, 206, 207, 208 are used by the control unit 209 for determining the weight of the particular fluid introduced into the line or collected.

In order to determine the amount of fluid released or collected in a particular bag or container the control unit 209 compares at regular intervals (the greater the flows the smaller the intervals) the actual weight of the container with the desired weight (which is a direct function of the desired flow for each pump and of the time interval between each control step ΔW = Q Δt).

The desired weight can be calculated as a function of the required flow (stored in a suitable storage unit of the computer) and of the time elapsed from the beginning of the treatment.

If the actual weight and the desired weight differ from each other, the control unit acts on the corresponding pump so as to reduce, and possibly cancel, said difference. In other words, during each cycle not an absolute weight variation, but only the variation in the time interval is taken into consideration to correct the latter.

The control unit takes into consideration variations in the difference starting from the last comparison, so as to avoid oscillations of the actual flow around the desired flow.

After the above description of the hydraulic circuit and of the possible working of the apparatus according to the invention incorporating said circuit, here is shown a detailed structure of the support element 4 according to the invention.

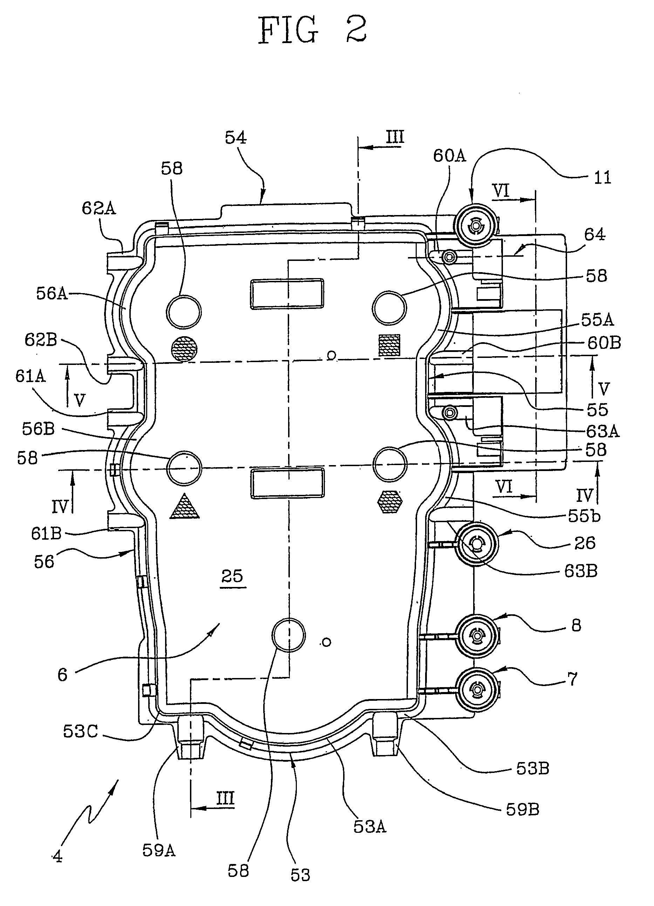

The support element as shown in the Figures 2 to 8a generally consists of a main body 6 and of a support structure 64 associated to said main body 6 and placed laterally with respect to the latter.

The main body 6 has a front wall 25 which is generally, though not necessarily, plane; then there is at least a peripheral wall 32 projecting away from the front wall 25 so as to define with the latter a housing compartment 33 designed to receive at least a portion of a fluid distribution circuit 15 to be associated to said support element.

As can be seen from Figure 2, the front wall 25 is delimited by a given number of sides 53, 54, 55 and 56, and the peripheral wall 32 projects away from each of said sides.

It should be noted that the sides referred to above are basically rectilinear and, generally, at least first sides 55, 56 and at least second sides 53, 54 can be identified, which are basically parallel and facing each other.

In other words, in a view from above the support element 4 has an approximately quadrangular shape and its front wall 25 is delimited by first opposite longer sides 55, 56 with a basically rectilinear development and having each two curved portions 55a, 55b; 56a, 56b whose cavities face their respective opposite side.

In further detail each of said curved portions 55a, 55b; 56a, 56b can be defined by an arc of circle.

Then there are second opposite shorter sides 53, 54, whose development is again basically rectilinear; at least one of said second sides 53, 54 has a curved portion 53a placed between two rectilinear lengths 53b, 53c, which has in its turn a cavity facing the opposite side.

Here again the curved portion 53a can be defined by an arc of circle.

As can be further noted by simply observing Figure 2, the arc of circle defining the curved portion 53a has a greater radius of curvature than the curved portions 55a, 55b, 56a, 56b defined on the first opposite longer sides 55, 56, as shall be better explained later.

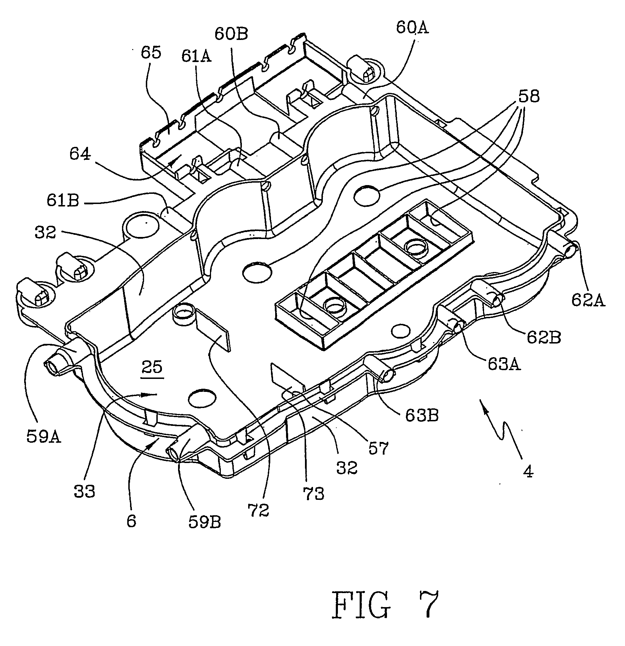

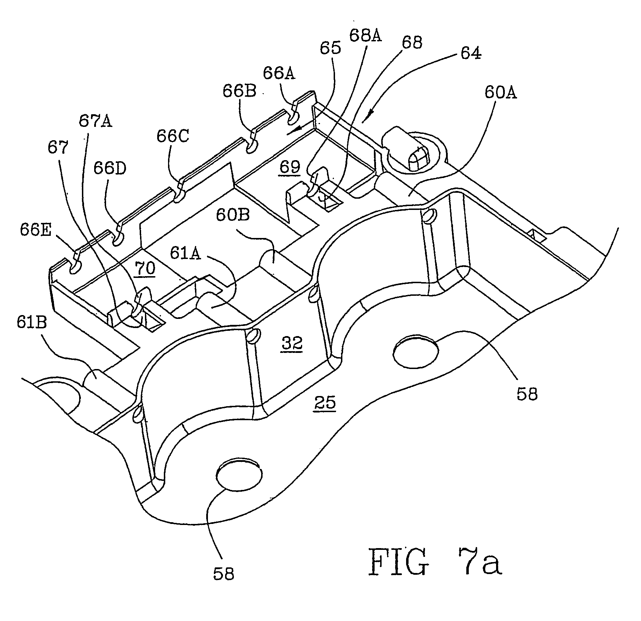

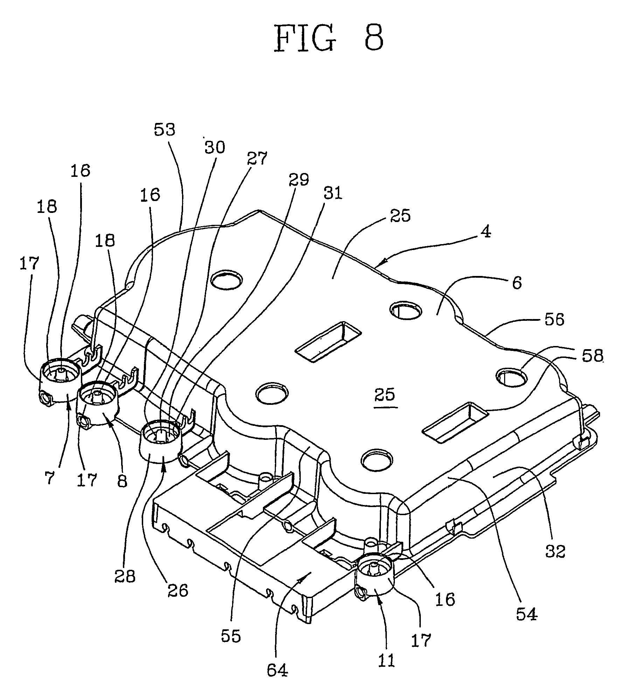

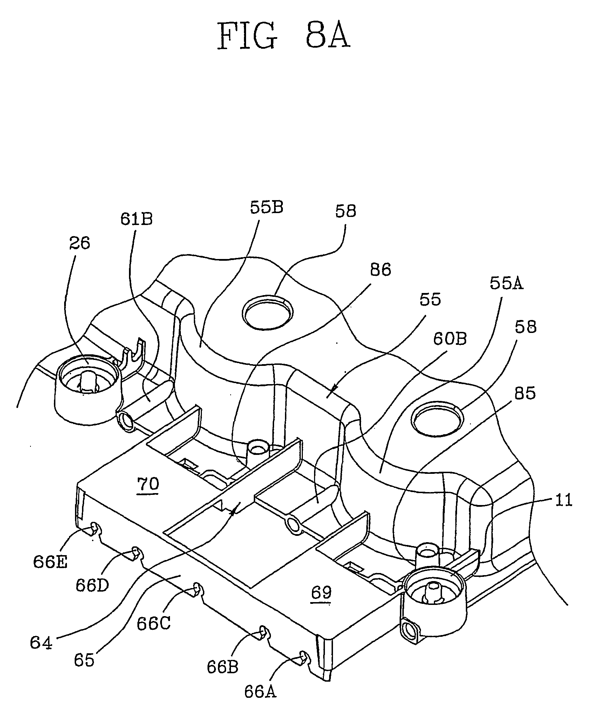

Examining now the peripheral wall 32 (see Figs. 7 and 8), it can be noted that it has at least a portion projecting away from each of the sides of the support element 4.

Generally, there will be at least one portion projecting from the first opposite sides 55, 56, and one projecting away from each of the second opposite sides 53, 54.

It is also evident that the peripheral wall 32 can also be discontinuous, i.e. it can have cavities or interruptions provided that it globally enables to define the aforesaid housing compartment 33.

The embodiment shown in Figures 7 and 8 is characterized in that the peripheral wall 32 projects away from all the sides of the front wall 25 and defines a basically continuous surface delimiting the housing compartment 33.

In other words, the housing compartment 33 has an access opening 57 without any kind of closing wall, which access opening is designed to face - when the support element 4 is being used - the extracorporeal blood treatment apparatus 2.

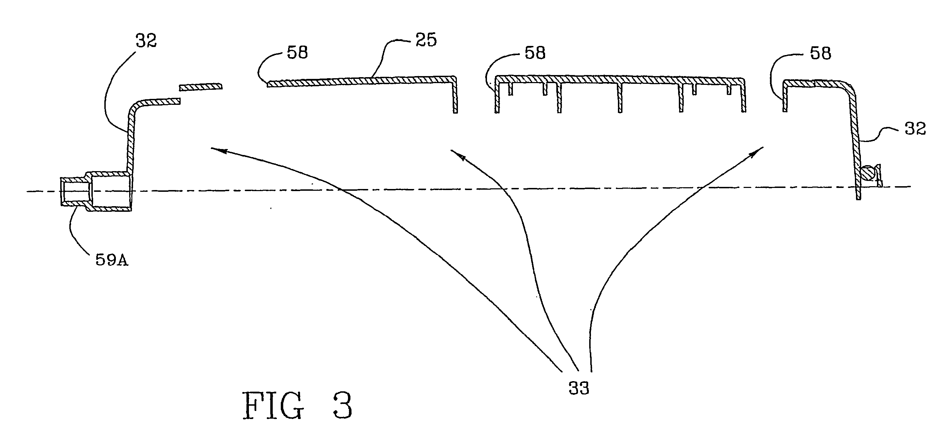

Moreover, from Figures 3, 4, 5 it can be inferred how sections according to a plane transversal with respect to the front surface, and in particular sections according to planes orthogonal to said front surface 25, show that the main body has a substantially C-shaped profile.

The peripheral wall 32 defines the two end lengths of said C, whereas the front wall 25 defines the intervening elongated length.

It should be noted how the front wall 25 and the peripheral wall 32 define a main body 6 having a box-shaped structure basically closed on five out of its six faces.

Said arrangement results in that, however sectioning the support element 4 according to two planes orthogonal one to the other and transversal to the front surface 25, the main body 6 will have C-shaped sections that are also orthogonal one to the other.

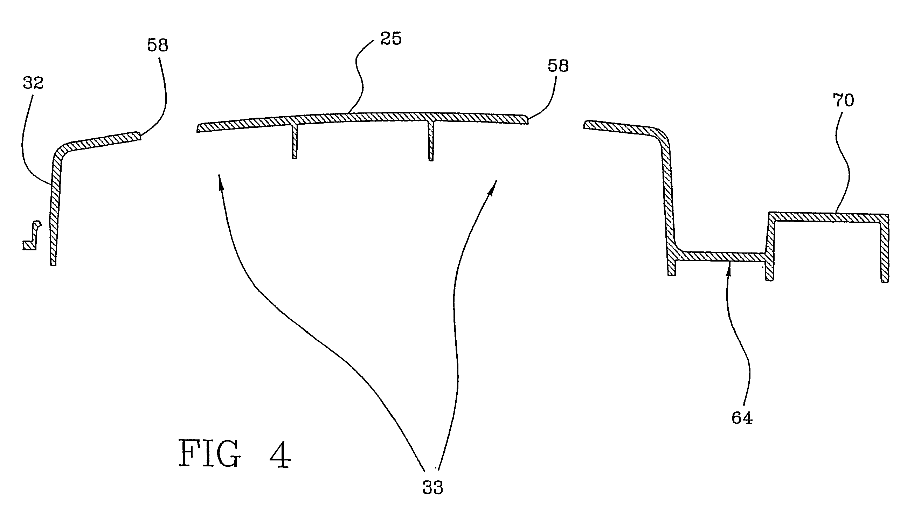

See in particular for instance the sections of Figures 3 and 4.

In still other words, the support element 4 comprises a front wall 25 which is able to connect opposite peripheral walls projecting in a basically perpendicular direction from said front wall 25.

As shown in Figures 2, 7 and 8, the front wall 25 has a given number of through openings 58 putting into communication the housing compartment 33 with the outside environment while the support element is being used.

Referring in particular to the figures described above, it can be noted that there is at least an opening 58 on each of the curved portions 53a, 55a, 55b, 56a and 56b and that said openings are defined by concentric round holes placed on the same axis as the respective arcs of circle defining the curved portions.

As far as the materials used are concerned, it should only be pointed out that the main body will be made of a stiff material, plastic for instance, which can protect the various tube lengths and/or elements therein contained.

It is also possible to carry out the whole support element or only a part of it with a material that is also transparent so as to obtain a visual access to the housing compartment 33.

Going into deeper structural details and referring in particular to Figure 7, it can be noted that there are several engagement connectors fastened to the respective sides of the box-shaped body.

In particular, there are at least a first and a second engagement connector 59a, 59a placed laterally with respect to the curvilinear length 53 of one of said second sides 53.

Said connectors shall be secured and generally carried out as one piece with said rectilinear lengths 53b and 53c.

There are also pairs of engagement connectors 60a, 60b, 61a, 61b, 62a, 62b, 63a, 63b respectively engaged near each of the curved portions 55a, 55b, 56a, 56b of the first longer sides 55, 56.

In other words, there will be two of said connectors placed exactly on opposite ends of each of the curved portions.

As in the case of the previous connectors, also the engagement connectors 60a, 60b, 61 a, 61b, 62a, 62b, 63a, 63b are carried out as one piece with the main body 6.

Furthermore, all the aforesaid connectors are fastened to the peripheral wall 32, for instance on a free edge of said peripheral wall.

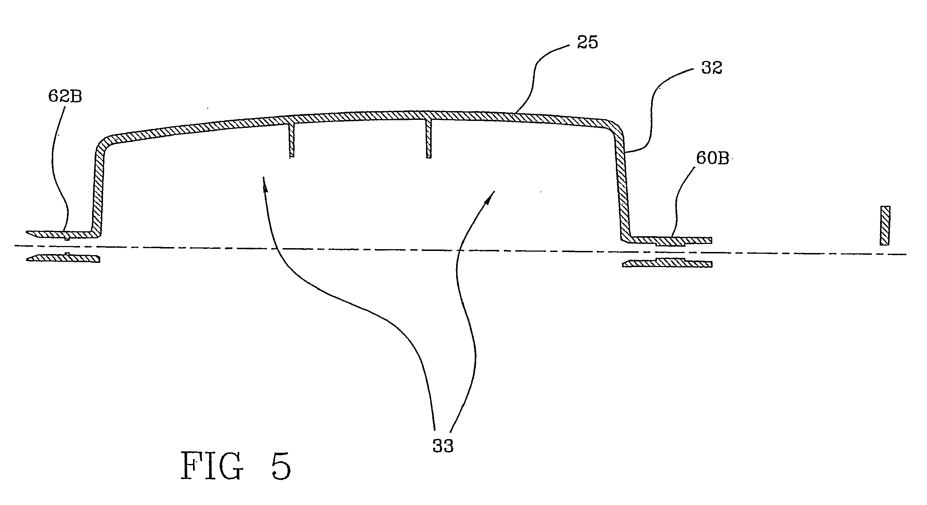

As can be seen in the section of Figure 5, each engagement connector defines a gap leading towards the housing compartment 33.

Referring now to Figures 7a and 8a, it can be noted how the support structure 64 associated to the main body 6 is positioned laterally with respect to the latter.

Also the support structure 64 is stiffly secured to the main body and will generally be carried out as one piece with the latter.

It should be pointed out that the support structure 64 is engaged to the main body 6 on one of the first longer sides 55, 56 and, in further detail, on the curved portions 55a, 55b of said fist longer side 55.

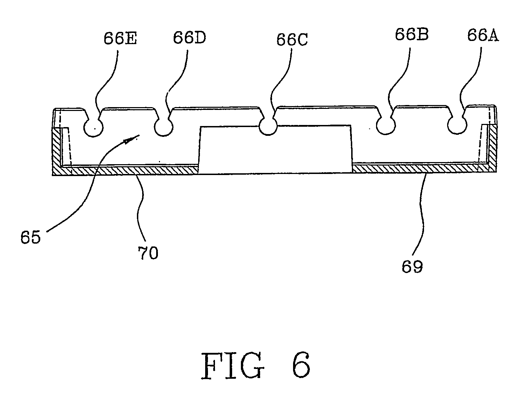

The support structure 64 is equipped with a positioning fin 65 (see again Figures 7a, 8a and the section of Figure 6), which has a given number of main seats 66a, 66b, 66c, 66d, 66e suitably placed so that respective tubes of the fluid distribution circuit 15 associated to the support element 4 can be engaged therein.

Referring to the relative position of the various components of the support structure 64, it can be noted how at least two, and generally three of said main seats 66a, 66c, 66d are placed on their respective engagement connector 60a, 60b, 61a located near the curved portions 55a, 55b of one of the first longer sides 55.

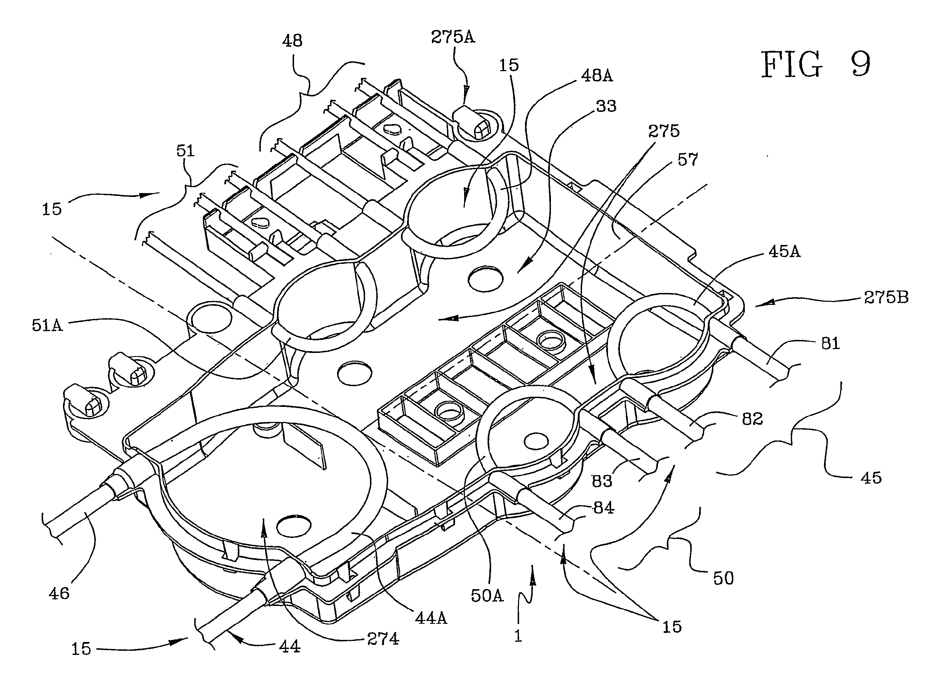

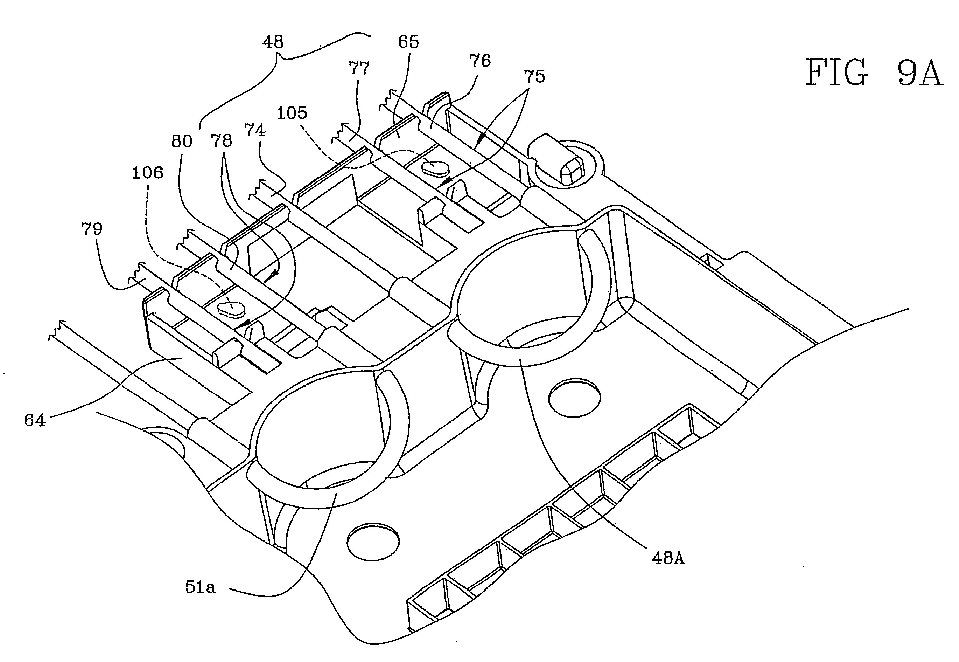

In other words, the three main seats 66a, 66c, 66d and their respective connectors 60a, 60b, 61a are positioned so as to receive parallel tube lengths (see to this end Figures 9 and 9a).

Going back to Figure 6 and to Figures 7a and 8a, it can be noted how the positioning fin 65 comprises two further main seats 66b and 66e and how also the support structure 64 is equipped with two auxiliary portions 67 and 68, each of them being provided with a respective auxiliary seat 67a, 68a so that the latter can cooperate with one another thus enabling the positioning of tube lengths parallel one to the other and generally parallel to those present on the three main seats and on the three engagement connectors referred to above (see again Figures 9 and 9a).

The support structure 64 then comprises at least a first covering wall 69 lying on a plane parallel to the plane of the front wall 25 so as to cover at least two parallel tube lengths in operating conditions in which the support element is engaged to the apparatus.

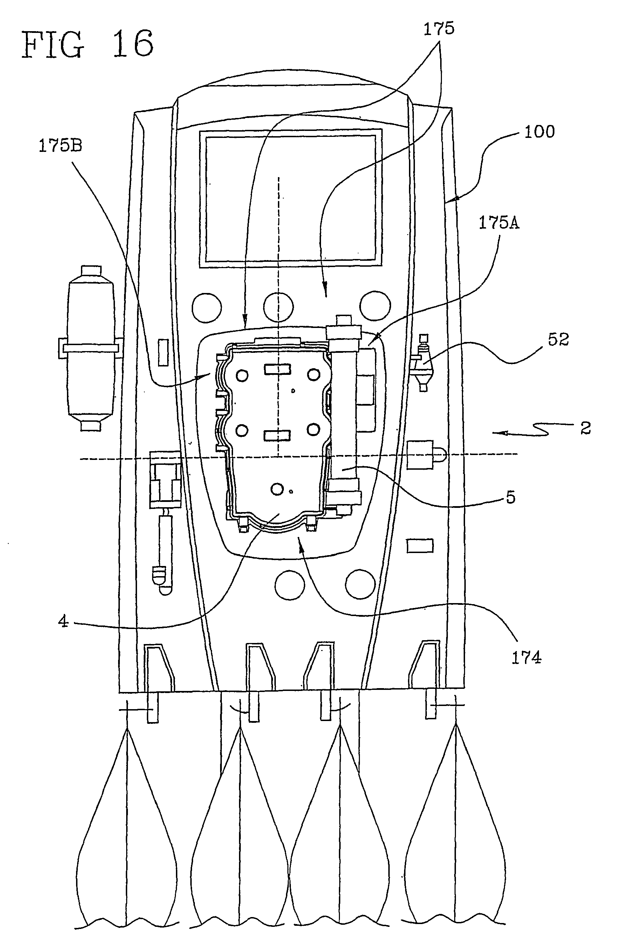

Compare to this end Figures 9 and 16.

In a wholly specular way the support structure 64 comprises at least a second covering wall 70 lying again on a plane parallel to the plane of the front wall 25 so as to cover at least two further parallel tube lengths when the support element is again in operating conditions.

Referring to Figure 8 it should then be pointed out that the support element 64 has a smaller height than - or at the most the same height as - the peripheral wall 32 of the main body.

This means that the support structure 64 has been designed so as not to increase the height of the whole support element.

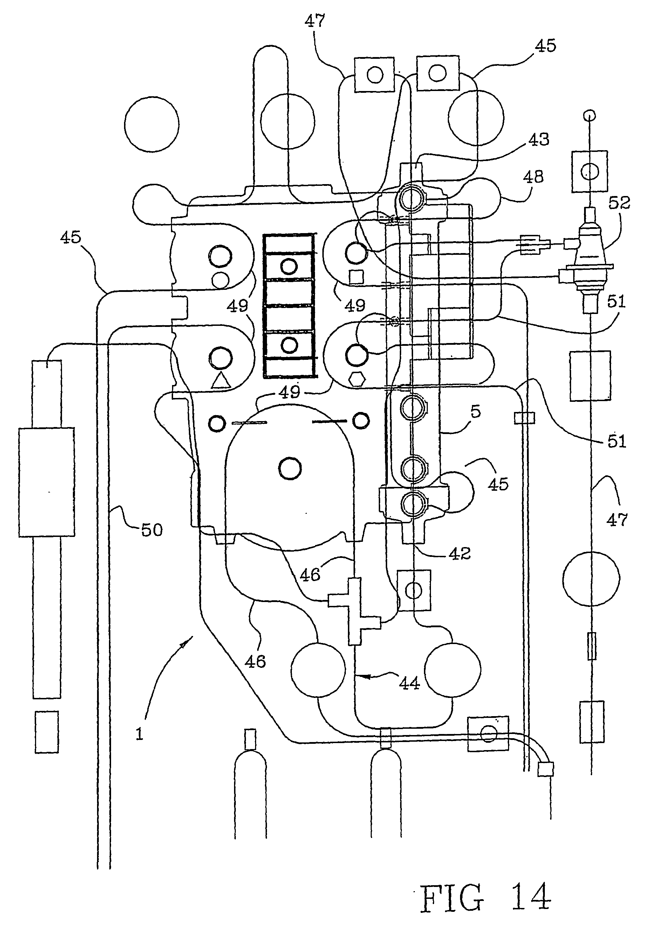

Referring now to Figure 7, further note at least one and in generally two positioning projections 72 and 73 associated to the main body 6 and designed to enable the correct positioning of a tube length to be associated to the support element as shall be better explained later (see anyway Figures 9 and 14).

Said first and second positioning projections 72, 73 are placed inside the housing compartment 33 and are generally associated (or also carried out as one piece) to the front wall 25.

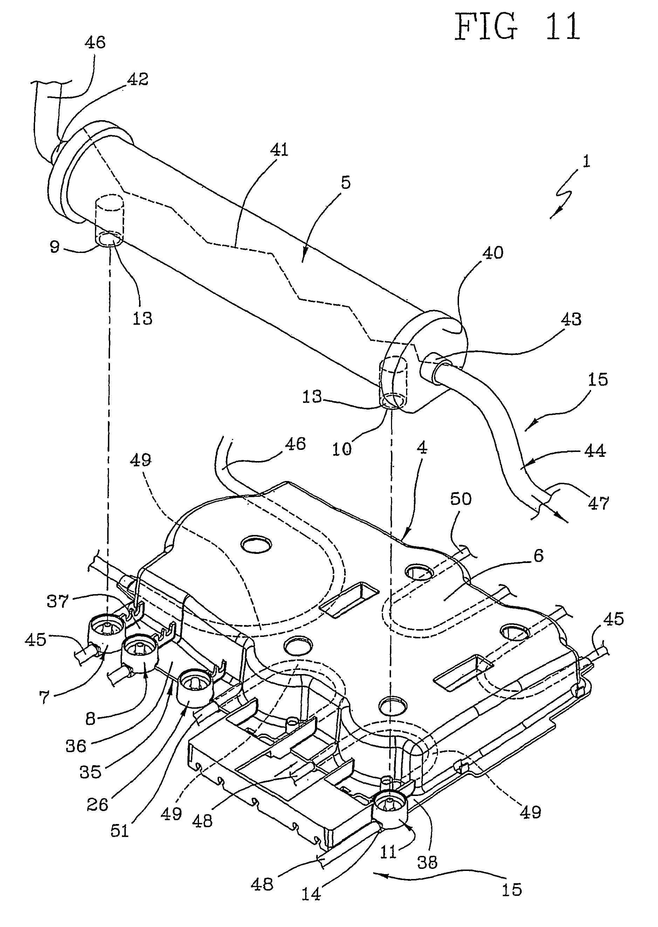

It should then be noted that the support element 4 comprises a main body 6 having at least a first and a second connector 7 and 8, spaced away from each other, in which corresponding counter-connectors 9 and 10 of the treatment unit 5 (see Figure 11) are engaged.

The blood treatment unit 5 can for instance be a plasma filter, a haemodialysis filter, a haemofiltration filter, a haemodiafiltration filter or another type of unit.

The first and second connector 7 and 8 are directly engaged to the main body 6; in the examples shown said connectors are made of stiff plastic material and as one piece with the main body.

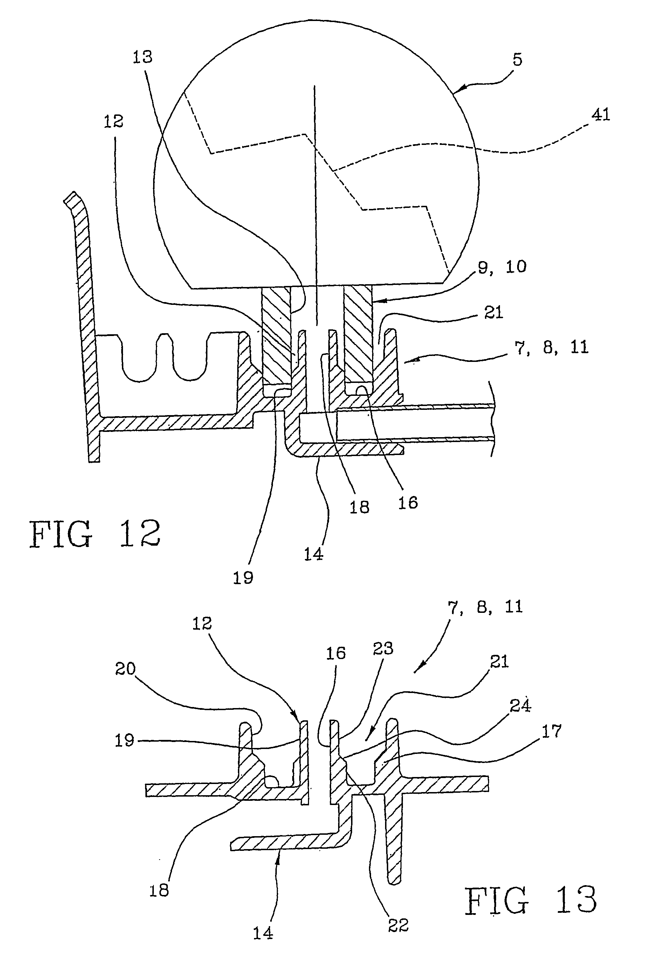

The support element 4 has a third connector 11 spaced away from the connectors 7 and 8 and engaged directly to the main body 6; in the examples shown also the third connector is made of stiff plastic material and as one piece with the main body; said three connectors define pairs of connectors having a differentiated central axis one with respect to the other for the engagement of corresponding pairs of counter-connectors associated to different blood treatment units that can be mounted onto the support element. Thus, one main body 6 can be used to carry out integrated modules with different features, thanks to the possibility of engaging treatment units 5 not only with different membranes but also with different global size and therefore with different central axis of the corresponding counter-connectors. Each of the connectors 7, 8, 11 referred to is a stiff support and defines a fluid passage having a first end portion 12, designed to be put into fluid communication with a corresponding channel 13 present in the respective counter-connector 9, 10 housed in the treatment unit 5 (see also the sections of Figs. 12 and 13); each connector 7, 8, 11 also has a second end portion 14, designed to be put into fluid communication with a fluid distribution circuit 15 to be associated to the main body 6. Going into further structural detail, each of said connectors 7, 8, 11 comprises a tubular channel 16 defining said first portion, a sealing collar 17 placed radially outside the tubular channel, and a connection wall 18 developing without interruptions between an outer side surface 19 of the tubular channel and an inner side surface 20 of said collar. In practice, the outer side surface of the tubular channel, the inner side surface of the sealing collar and the connection wall define a ring-shaped engagement seat 21, whose bottom is delimited by the connection wall, shaped so that a corresponding counter-connector of the treatment unit can be engaged therein. The tubular channel 16 is arranged coaxially with respect to the sealing collar 17, and both turn around a common symmetry axis. The ring-shaped seat 21 has an increasing radial size getting away from the bottom wall and comprises a first zone 22 near the bottom, having a constant radial size, a second zone 23, distal with respect to the bottom and with a constant radial size greater than the radial size of the first zone, and a third zone 24 between the first and the second zone, having a progressively increasing size getting away from the bottom wall 18. The tubular channel and the sealing collar of each connector 7, 8, 11 project parallel one to the other from the main body 6, so as to define one direction of coupling with the corresponding counter-connectors of a treatment unit 5. In the examples of embodiment shown the various connectors have a symmetry axis that is basically orthogonal with respect to a front surface 25 of the support element 4.

The support element shown also comprises a fourth connector 26 spaced away from said first, second and third connector; the fourth connector is also connected directly to the support element. In the example shown the fourth connectors is made of stiff plastic material and as one piece with the main body 6 and defines with at least one of the other connectors a further pair of counter-connectors associated to a blood treatment unit to be mounted onto the support element. The fourth connector comprises a central cylindrical positioning body 27, a sealing collar 28 placed radially outside the cylindrical body, and a connection or bottom wall 29 developing without interruptions between an outer side surface 30 of the cylindrical body and an inner side surface 31 of said collar. In practice, said fourth connector defines an engagement and flow-closing body for a counter-connector of the treatment unit 5. As shown in Figures 11, 12 and 13, the various connectors are made of stiff material so as to define a mechanical support of the treatment unit and, if needed, so as to define a passage or a blocking member for the fluid getting through the counter-connectors 9, 10. The four connectors that are present in the support element are aligned one with respect to the other and arranged on one side of said main body. More to the point, the main body of the element shown defines the aforesaid housing compartment 33, which can house at least a portion of the fluid distribution circuit 15 designed to be associated to the support element 4. The housing seat has an open side 57 ensuring a suitable fitting and positioning of the integrated module 1 onto the apparatus 2, as shall be disclosed later in further detail. The support element then has an auxiliary structure 35 extending laterally and outside with respect to the operating seat from a base zone 36 of the peripheral wall 32. The four connectors come out from the auxiliary structure the first, second and fourth 7, 8, 26 are placed one beside the other and are arranged on a first end zone 37 of the auxiliary structure, whereas the third connector 11 is placed on a second end zone 38 placed opposite the first one.

A support element according to the invention can be suitably used for carrying out an integrated module, such as for instance the one shown in Figures 9-11, in which the support element of Figures 2-8 is used by way of example. As can be seen, the treatment unit 5 is fastened to the support element 4 on at least the pair of connectors; the treatment unit comprises a housing body 40, at least a semipermeable membrane 41 (for instance with parallel hollow fibers or with plates) operating inside the housing body and defining a first chamber and a second chamber; a first and a second counter-connector are associated to the housing body and secured to their respective connectors housed by the main body 6 (see for instance Figure 11).

The first and second counter-connector 9, 10 have a tubular shape and are put into fluid communication with the second chamber of the treatment unit and with respective end portions 12 of said connectors. The treatment unit then has an access port 42 leading to the first chamber, and at least an exit port 43 from said first chamber, for the connection with an extracorporeal circulation line 44 for blood or another physiological fluid.

A fluid distribution circuit 15 is engaged to the support element 4 and cooperates with the treatment unit 5.

In further detail said circuit comprises the aforesaid blood line 44, which is fastened to the support element 4 on one of the second sides 53, 54 and has the curved portion 53a.

The blood line 44 is secured to the support element so as to define at least a tube length basically arranged as a U 44a with respect to said support element.

Said arrangement is related to the fact of enabling the cooperation between said tube length 44a and a respective pump 3 a while assembling the integrated module onto the apparatus 2.

As can then be inferred from the appended figures, the U-shaped tube length 44 extends inside with respect to the peripheral wall 32 of the support element 4.

The positioning projections 72, 73 previously described act on the U-shaped tube length 44a so as to keep its correct position.

As can be inferred from Figures 1 and 9, the length 44a of the blood line 44 secured to the support element is defined by the intake branch 46.

The distribution circuit 15 then has the aforesaid inlet line 48 supplying fresh dialysis liquid.

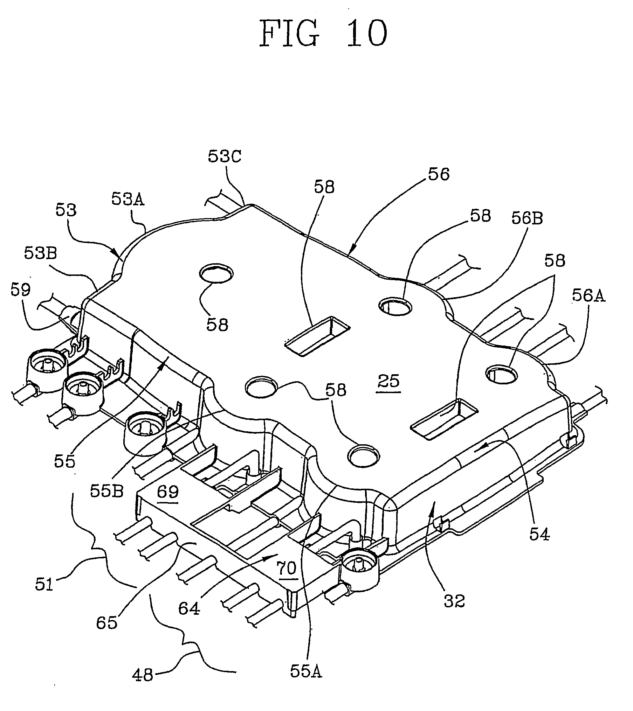

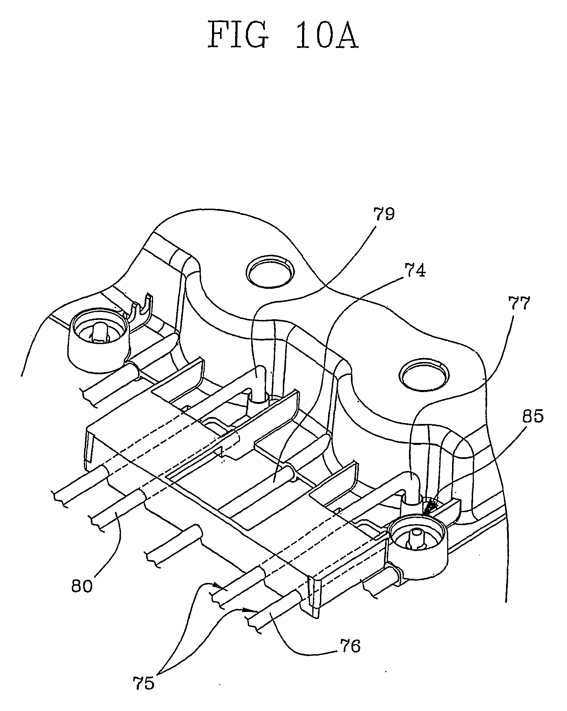

Said line is fastened to the support element on one of the first longer opposite sides 55, 56, as can be seen in Figures 9, 9a, 10 and 10a.

Also the inlet line 48 is secured to the support element so as to define at least a tube length basically arranged as a U 48a with respect to said support element.

Also the tube length 48 is designed to cooperate with a respective pump 3c and is placed inside with respect to the peripheral wall 32 of the support element.

Referring to Figure 9a it can be noted how the inlet line 48 is fastened to the main body 6 on the support structure 64, and how at least an inlet length 74 of the inlet line 48 is engaged into a main seat 66c of the positioning fin 65, as well as to the respective engagement connector 60b.

Analogously, at least an outlet length 75 of the inlet line is engaged into a main seat 66a of the positioning fin 65 and to the respective engagement connector 60a.

When engaged, the respective connectors and inlet and outlet lengths 74 and 75 are placed in a rectilinear arrangement and are parallel one to the other (see Figure 9a).

As can further be seen (see in particular Figure 10a), the outlet length 5 has a branching 85 splitting up into intake branch 76 conveying the fluid to the blood treatment unit 5, and into infusion branch 77 conveying the fluid into the blood line 44.

Said branching 85 is defined on the engagement connector 60a having a T shape with an inlet and two outlets.

Also the infusion branch 77 is secured to a main seat 66b and to an auxiliary seat 77a.

The infusion branch 77 and the intake branch 76, when engaged to the support structure 64, are placed in a rectilinear arrangement and are parallel one to the other.

The fluid distribution circuitry 15 then comprises at least the infusion line 51, which is also fastened on one of the first longer opposite sides 55, 56.

Said infusion line defines a tube length arranged as a U 51a with respect to said support element 4, so as to be able to cooperate, when in use, with a respective pump 3d.

Also the U-shaped tube length 51a extends inside with respect to the peripheral wall 32 of the support element.

Also the infusion line is secured on the support structure 64 and at least an outlet length 78 of the infusion line 71 is engaged into a main seat 66d of the positioning fin 65 and to its respective engagement connector 61a as shown in the appended figures.

In a wholly specular way to the intake line, the outlet length 78 has a branching 86 splitting up into pre-infusion branch 79 conveying the fluid to an intake branch 46 of the blood line 44, and into post-infusion branch 80 conveying the fluid to a blood return branch 47 of the blood line.

Here again there is an engagement connector 61a having a T shape so that the branching 86 into pre-infusion branch 79 and into post-infusion branch 80 is defined exactly by said connector 80.

The pre-infusion branch 79 is then fastened to an auxiliary seat 68a and to a further main seat 66e of the positioning fin 65.

When engaged to the support structure, said two branches 79 and 80 are placed in a rectilinear arrangement and are parallel to one another.

The fluid distribution circuit 15 then has the discharge line 45 secured to the support element also on one of said first longer sides 55, 56.

Said discharge line 55 defines at least a tube length arranged as a U 45a with respect to the support element, which tube length is also designed to cooperate with a respective pump 3b and extending inside with respect to the peripheral wall 32 of the support element.

The discharge line 45 is secured to the main body 6 on an opposite side with respect to the support structure 64 and the respective inlet length 81 and outlet length 82 are engaged into corresponding engagement connectors 62b, 62a.

Eventually, the distribution circuit 15 has the auxiliary pre-infusion line 50.

The latter is fastened to the support element 4 on one of said first longer sides 55, 56 so as to define at least a further tube length arranged as a U 50a with respect to said support element.

Also the tube length 50a is designed to cooperate, when in use, with a respective pump 3e and extends inside with respect to the peripheral wall 32 of the support element.

In other words, the housing compartment 33 is designed to house all U-shaped tube lengths of the various lines of the distribution circuitry 15.

The auxiliary pre-infusion line 50 is secured to the main body on an opposite side with respect to the support structure 64 and the respective inlet length 83 and outlet length 84 are engaged to engagement connectors 63b, 63a.

It should then be pointed out that the particular shape of the peripheral wall 32 of the support element 4 defining the arched portions and the peculiar position of the engagement connectors of the various tubes result in that the length of every free U-shaped tube portion 44a, 45a, 48a, 50a, 51a is smaller than or the same as πR + 2R, where R is the radius of curvature of the tube length.

The peculiar shape of the integrated module is such that the free lengths within the housing compartment 33 are as short as possible in accordance with the radial sizes of the respective pumps which have to generate the flow within said tubes.

It should then be noted how the U-shaped tube length 44a of the blood line is longer than the tube lengths 45a, 48a, 50a, 51a defined by the further fluid lines having indeed a longer radius of curvature.

Moreover, the tube length of the blood line can be carried out, if needed, with materials differing from those of other tubes and/or it can have sections for the passage of fluid differing from the other tubes.

From the point of view of the geometrical position of the various tube lengths on the support element note the following.

First of all, the support element can be ideally divided into several zones comprising a first zone 274 secured to the portion of the blood line 44 which, in operating conditions of the module 1 engaged to the apparatus 2, shall be defined by the lower zone of said module.

Therefore, there will be a second zone 275 opposite the first zone, to which all the further fluid lines 45, 48, 50 and 51 are secured.

Said second zone consists in its turn of at least two ideal half-parts placed side by side 275a, 275b.

The tube length 45a of the discharge line 45 and the tube length 50a of the auxiliary pre-infusion line 50 will be fastened to the second half-part 275b.

Conversely, the tube length 48 of the intake line and the tube length 51a of the infusion line are fastened to the first half-part 275a. Said splitting into first and second zone 274, 275 and the two half-parts 275a, 275b of the second zone have been ideally shown in Figure 9 by means of hatched lines.

As can be noted, the first and second half-part 275a, 275b of the second zone 275 are reciprocally placed side by side and generally perfectly symmetrical to a longitudinal axis of the main body 6. Should the first zone 274 be geometrically delimited, it could be defined as the area limited by at least one of the second sides 53 (having the curved portion and to which the blood line is secured) and by about half the length of the first opposite longer sides 55 and 56 near the second side 53.

Analogously, the second zone 275 is partly delimited by one of said second sides 54 which has no curve and by a portion of the first opposite longer sides 55 and 56 near said second side 54.

The assembly process of an integrated fluid treatment module comprises the stage of installation of a support element 4, for instance as shown in Figures 2-8, and a treatment unit 5 to be coupled to the support element. Then the blood treatment unit is fastened to the support element. Eventually, a fluid distribution circuit 15 is associated to the support element and to the treatment unit so as to create the necessary lines for blood circulation, discharge, infusion of possible substitution liquids, dialysis. Note that the connection of the distribution circuit to the treatment unit can be before, simultaneous to or follow the stage in which the circuitry is fastened to the support element. The stage in which the treatment unit is fastened to the support element comprises sub-stages in which a pair of connectors to which the counter-connectors 9, 10 housed by the blood treatment unit are to be fastened are chosen, in which a given amount of glue, normally based on a polymer resin, is placed in the ring-shaped seats 21 of each connector chosen, in which each counter-connector is at least partially fitted into its respective ring-shaped seat so as to obtain a mechanical blocking and a liquid-sealing coupling. Note that during said fitting stage at least a portion of the glue placed in the ring-shaped seat reaches the second zone 23 of said ring-shaped seat. At the end of said stage in which the counter-connector is fitted into its respective ring-shaped seat, the volume of glue previously placed plus the volume of the portion of counter-connector housed within the ring-shaped seat is smaller than the total volume of said ring-shaped seat. It is thus avoided that glue migrates towards the tubular channel 16 causing its partial or total occlusion.

The stage in which a fluid distribution circuit 15 is associated to the support element 4 and to the treatment unit 5 comprises in its turn the sub-stages in which an end portion of a discharge line 45 for a waste fluid is fastened fluid-sealingly with the second end portion 14 of one of said connectors, and in which an end portion of an intake line 48 for fresh dialysis liquid is fastened sealingly with the second end portion of another of said connectors. Said stage of association of the distribution circuit also comprises the sealing fastening of an end portion of a blood suction branch 46 with the inlet port to the first chamber, and an end portion of a blood return line 47 with the exit port from said first chamber. The fastening of the various end portions referred to above can take place by gluing, by forcing or by hot coupling.

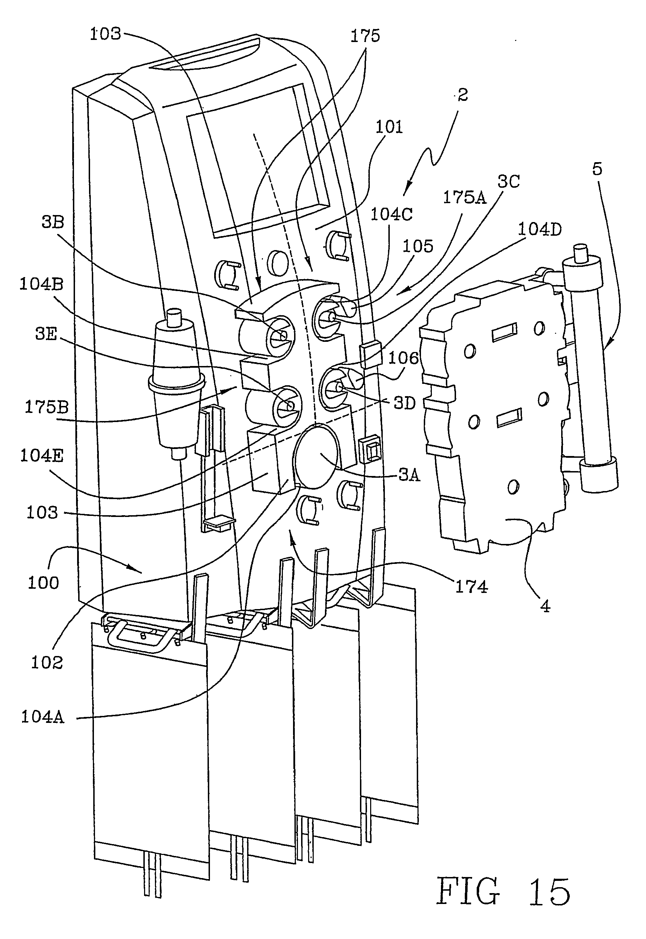

Granted the above, it should be noted that the integrated module according to the present invention is designed to be used on an extracorporeal blood treatment apparatus 2 as shown in Figures 15 and 16.

In particular, said apparatus 2 comprises a body 100 provided on its front surface 101 with a given number of peristaltic pumps 3a, 3b, 3c, 3d, 3e designed to cooperate with the respective U-shaped tube lengths defined on the integrated module.

As can be noted from Figure 15, the apparatus body 11 has a guiding and positioning projection 102 protruding from the surface 101, which is exactly counter-shaped to the peripheral wall 32 of the support element to which it should be coupled.

In other words, the guiding and positioning projection 102 has a side surface 103 which, when engaged to the integrated module, is delimited by the peripheral wall 32.

Also the peristaltic pumps protrude from the surface 101 of the apparatus body 100 and at least a part of their side surface is counter-shaped to the peripheral wall 32 of the support element.

In particular, it is exactly the curved portions defined by the curved lengths of the front wall 25 which are designed to couple with the protruding side portions of the pumps 3.

The protruding peristaltic pumps and the guiding and positioning projection 102 define together suitable seats 104a, 104b, 104c, 104d and 104e taking a basically semicircular or U shape and designed to receive the corresponding U-shaped tube lengths 44a, 45a, 48a, 50a, 51a.

Analogously to what has been described for the integrated module 1, also on the front wall of the apparatus a given number of zones can be defined, and in particular two zones 174, 175 in which the first zone 174 comprises the blood pump 3a, whereas the second zone 175 comprises the other pumps 3b, 3c, 3d and 3e.

The second zone 175 comprises at least two half-parts placed side by side 175a, 175b; the intake pump 3c and the infusion pump 3d are placed in said first half-part whereas the auxiliary pre-infusion pump 3e and the suction pump 3b are placed in the second half-part.

Here again the first and second half-part are specularly symmetrical and placed side by side on the front wall of the apparatus and above the first zone 174.

Eventually, it should be noted that there is at least a first moving element 105 and a second moving element 106 that are substantially identical and housed directly by the apparatus body; the latter are designed to act respectively on the infusion branch 77 and/or on the intake branch 76 (the first moving element), and on the pre-infusion branch 79 and/or on the post-infusion branch 80 (the second moving element 106). In particular, the selecting means 97 and 203 previously described can comprise said moving elements 105, 106 designed to be controlled by the CPU 209 so as to selectively determine the blocking or passage of fluid in either branch.

In order to cooperate with said moving elements the integrated module is equipped with the support structure with said infusion, intake, post-infusion and pre-infusion branches, which are all parallel to one another.

The invention has important advantages.

First of all, the present invention allows to obtain an integrated module for apparatus for extracorporeal blood treatment with an optimal arrangement of the various tube lengths of fluid lines.

The division of said module into two opposite zones allows to engage to one of the shorter sides a U-shaped tube length of a blood circuit with greater size than the U-shaped tube lengths of the other fluid lines, thus enabling the use of peristaltic pumps with greater size, which can allow higher flow rates and also, since longer tube lengths are used, less damages of the tube length on which the pump acts.

Moreover, the particular arrangement of the intake line for fresh dialysis liquid on the blood treatment element and on the post-infusion zone enables to minimize the length of the inlet portion of said intake line, thus minimizing the amount of fresh dialysis fluid to be wasted.

The same applies also to the pre-/post-infusion lines, which is also placed on the pre- and post-infusion zones and enables to minimize the lengths of the various branches.

It is evident that said positioning is extremely advantageous in intensive therapy apparatuss in which all biological fluids are contained in bags with limited volume.

Eventually, it should be pointed out that the presence of five peristaltic pumps on the apparatus and of corresponding U-shaped tube lengths on the integrated module enables the use of another line, in particular of a pre-infusion line, so as to allow the introduction, for instance, of topical anticoagulants without limiting pre- and post-infusion possibilities.

Finally, the use of a particular support element that is open on one side and defines a basically box-shaped body enables an optimal protection of the tube lengths of the respective peristaltic pumps when the unit is operating.

## Claims

1. Integrated fluid treatment module comprising:
- a support element (4);
- a fluid distribution circuitry (15) associated to the support element (4) and comprising:
● at least a blood line (44), at least a portion of said blood line being fastened to the support element (4) and defining at least a U-shaped tube length (44a) with respect to said support element, said tube length (44a) being designed to cooperate with a respective pump (3a);
● further fluid lines secured to the support element, each of them defining at least a U-shaped tube length (45a, 48a, 50a, 51a) with respect to said support element, each tube length being designed to cooperate with a respective pump (3a, 3b, 3c, 3d, 3e), wherein
the support element (4) has a first zone (274) to which the portion of the blood line (44) is fastened, and at least a second zone (275) opposite said first zone, the other fluid lines (45, 48, 50, 51) all being fastened on said second zone;
the U-shaped tube length (44a) of the blood line (44) is longer than the tube lengths (45a, 48a, 50a, 51 a) defined by the other fluid lines;
the U-shaped tube length (44a) of the blood line has a greater radius of curvature than the tube lengths defined by the other fluid lines;
the fluid distribution circuitry (15) comprises at least a discharge line (45) for a waste fluid, said discharge line (45) for a waste fluid being secured to the support element (4) and defining at least a tube length arranged as a U (45a) with respect to said support element, said U-shaped tube length (45a) being designed to cooperate with a respective pump (3b);
the fluid distribution circuitry (45) comprises at least an intake line (48) for fresh dialysis liquid, the intake line (48) for fresh dialysis liquid being fastened to the support element (4) and defining at least a tube length arranged as a U (48a) with respect to said support element, said tube length (48a) of the liquid intake line being designed to cooperate, when in use, with a respective pump (3c);
the module further comprises at least a fluid infusion line (51), the fluid infusion line (51) being fastened to the support line (4) and defining at least a tube length arranged as a U (51 a) with respect to said support element, said fluid infusion line (51) being designed to cooperate, when in use, with a respective pump (3d);
the module further comprises at least an auxiliary pre-infusion line (50), the auxiliary pre-infusion line (50) being fastened to the support element (4) and defining at least a tube length arranged as a U (50a) with respect to said support element, said auxiliary pre-infusion line (50) being designed to cooperate with a respective pump (3e);
the support element (4) comprises a main body (6) having a front wall (25) and at least a periperal wall (32) projecting away from said front wall (25), said front wall (25) and said peripheral wall (32) defining a housing compartment (33);
the front wall (25) comprises at least first sides (55, 56) and second sides (53,54) basically parallel and reciprocally facing;
the front wall is delimited by first opposite longer sides (55,56) with a basically rectilinear development, each having two curved portions (55a, 55b, 56a, 56b) whose cavity faces its respective opposite side;
the tube length (48a) of the intake line (48) for fresh dialysis liquid is fastened to one of said first longer sides (55,56);
the tube length (45a) of the discharge line (45) for a waste fluid is fastened to one of said first longer sides (55,56);
the tube length (51 a) of the fluid infusion line (51) is fastened to one of said first longer sides (55,56);
the tube length (50a) of the auxiliary pre-infusion line (50) is fastened to one of said first longer sides (55,56);
the U-shaped tube lengths (44a, 45a, 48a, 50a, 51a) extend inside with respect to the peripheral wall (32) of the support element (4).

2. Module according to claim 1, **characterized in that** said second zone (275) comprises at least two half-parts placed side by side (275a, 275b), at least the tube length of the discharge line (45) being fastened to the second half-part (275b).

3. Module according to claim 1 or 2, **characterized in that** said second zone (275) comprises at least two half-parts placed side by side (275a, 275b), at least the tube length (48a) of the intake line (48) for fresh liquid being fastened to the first half-part (275a).

4. Module according to any of the preceding claims, **characterized in that** said second zone (275) comprises at least two half-parts placed side by side (275a, 275b), at least the tube length (51 a) of the infusion line (51) being fastened to the first half-part (275a).

5. Module according to any of the preceding claims, **characterized in that** said second zone (275) comprises at least two half-parts placed side by side (275a, 275b), at least the tube length (50a) of the auxiliary pre-infusion line (50) being fastened to the second half-part (275b).

6. Module according to any of the preceding claims, **characterized in that** the front wall is delimited by second opposite shorter sides (53, 54) with a basically rectilinear development.

7. Module according to claim 6, **characterized in that** at least one of said second sides (53) has a curved portion (53a) placed between two rectilinear lengths (53b, 53c), the cavity of said curved portion facing the opposite side.

8. Module according to claim 7, **characterized in that** the first zone (274) is partly delimited by at least one of said second sides (53) having the curved portion (53a) and by a portion of the first opposite longer sides (55, 56) beside said second side (54) having the curved portion.

9. Module according to claim 7, **characterized in that** the second zone (275) is partly delimited by one of said second sides (54) without the curved portion and by a portion of the first opposite longer sides (55, 56) beside said second side (54) without the curved portion.

10. Module according to any of the preceding claims, **characterized in that**, when the module is associated to an extracorporeal blood treatment apparatus (2), the first zone (274) of the support element (4) is placed below the second zone (275) of the support element.

11. Module according to any of the claims 2 to 5, **characterized in that**, when the module (1) is associated to an extracorporeal blood treatment apparatus (2), the first and second half-part (275a, 275b) of the second zone (275) of the support element (4) are placed side by side.

12. Module according to claim 7, **characterized in that** the tube length (44a) of the blood line is fastened to said second side (53) having the curved portion (53a).

13. Module according to claim 7, **characterized in that** one of said second sides (54) without the curved portion has no tube length directly fastened thereto.

14. Module according to any of the preceding claims, **characterized in that** a length of every U-shaped tube portion (44a, 45a, 48a, 50a, 51a) is smaller than or the same as (πR + 2R), where R is the respective radius of curvature of the tube length.

15. Module according to any of the preceding claims, **characterized in that** it further comprises a support structure (64) associated to the main body (6) and placed laterally with respect to the latter.

16. Module according to claim 15, **characterized in that** said support structure (64) is engaged to the main body (6) on one of said longer sides (55, 56).

17. Module according to claim 16, **characterized in that** the support structure (64) is engaged to the main body (6) on said curved portions (55a, 55b, 56a, 56b) of one of said first longer sides (55, 56).

18. Module according to any of the preceding claims, **characterized in that** it further comprises at least a blood treatment unit (5) engaged on the support element (4).

19. Module according to any of the preceding claims, **characterized in that** said support element (4) comprises at least a first and at least a second connector (7, 8) associated
to the main body (6) and spaced away one from the other, said first and said second connector (7, 8) being designed to receive by way of engagement corresponding counter-connectors (9, 10) of a blood treatment unit to be mounted onto the support element.

20. Module according to claims 18 and 19, **characterized in that** said treatment unit (5) comprises:
- a housing body (40);
- at least a semipermeable membrane (41) operating inside the housing body defining a first and a second chamber;
- a first and a second counter-connector (9, 10) associated to the housing body (40) and fastened to respective connectors (7, 8) associated to the main body (6), at least one of said first and second counter-connector (9, 10) being put Into fluid communication with the second chamber of the treatment unit and with respective first end portions of said connectors;
- at least an access port (42) to said first chamber; and
- at least an exit port (43) from said first chamber.

21. Module according to claims 15 and 19, **characterized In that** the connectors (7, 8) and the support structure (64) are positioned laterally with respect to the main body (6) on one of said longer sides (55, 56).

22. Module according to any of the claims 2 to 5, **characterized in that** the first and second half-part (275a, 275b) are specularly symmetrical with respect to a longitudinal axis of the main body (6).

23. Apparatus for extracorporeal blood treatment destined to receive an integrated module according to any one of the preceding claims, the apparatus comprising a body (100) having a surface (101), a guiding and positioning projection (102) protruding from the surface (101) and being exactly counter-shaped to a peripheral wall (32) of a support element to which It has to be coupled, a given number of pumps (3a, 3b, 3c, 3d, 3e) protruding from the surface (101) and designed to cooperate with a suitable fluid distribution circuitry (15) of the integrated module to be associated to the apparatus, at least a part of a side surface of the pumps (3a, 3b, 3c, 3d, 3e) is counter-shaped to the peripheral wall (32) of the support element, the protruding pumps and the guiding and positioning projection (102) define together seats (104a, 104b, 104c, 104d and 104e) taking a basically semicircular or U shape and designed to receive the corresponding U-shaped tube lengths (44a, 45a, 48a, 50a) of the fluid distribution circuitry (15) of the integrated module, wherein
at least one of said pumps being a blood pump (3a) designed to cooperate with a respective blood line (44) of the distribution circuitry (15),
at least one of said pumps is a feeding pump (3c) and is designed to cooperate with a respective feeding line (48) for fresh dialysis liquid of the distribution circuitry (15),
at least one of said pumps is a suction pump (3b) designed to cooperate with a respective discharge line (45) of the distribution circuitry (15),
at least one of said pumps is an infusion pump (3d) designed to cooperate with a respective infusion line (51) of the distribution circuitry (15),
at least one of said pumps is an auxiliary pre-infusion pump (3e) designed to cooperate with a respective auxiliary pre-infusion line (50) of the distribution circuitry (15), the apparatus body (100) defining on its surface (101) a first zone (174) having said blood pump (3a) and at least a second zone (175) opposite said first zone and having the other pumps (3b, 3c, 3d, 3e).

24. Apparatus according to claim 23, **characterized in that** said second zone (175) comprises at least two half-parts place side by side (175a, 175b), the feeding pump (3c) being placed in said first half-part (175a).

25. Apparatus according to any of the claims 23 and 24, **characterized in that** in operating conditions the first zone (174) of the apparatus body (100) is placed below the second zone (175) of said body.

26. Apparatus according to claim 24, **characterized In that** in operating conditions the first and second half-part (175a, 175b) of the second zone (175) of the apparatus body (100) are placed side by side.

27. Apparatus according to claim 24, **characterized in that** said first and second half-part (175a, 175b) are specularly symmetrical.

28. Apparatus according to any of the claims 23 to 27, **characterized in that** said second zone (175) comprises at least two half-parts place side by side (175a, 175b), the suction pump (3b) being placed in said second half-part (175b).

29. Apparatus according to any of the claims 23 to 28, **characterized in that** said second zone (175) comprises at least two half-parts place side by side (175a, 175b), the infusion pump (3d) being placed in said first half-part (175a).

30. Apparatus according to any of the claims 23 to 29, **characterized In that** said second zone (175) comprises at least two half-parts place side by side (175a, 175b), the auxiliary pro-infusion pump (3e) being placed in said second half-part (175b).

## Patentansprüche

1. Integriertes Modul zur Blutbehandlung, umfassend:
- ein Trägerelement (4);
- einen Kreislauf zur Fluidverteilung (15), der mit dem Trägerelement (4) verbunden ist und umfasst:
• mindestens eine Blutleitung (44), wobei mindestens ein Teil der Blutleitung am Trägerelement (4) befestigt ist und mindestens einen U-förmigen Schlauchabschnitt (44a) am Trägerelement definiert, wobei der Schlauchabschnitt (44a) dafür vorgesehen ist, mit einer entsprechenden Pumpe (3a) zusammenzuarbeiten;
• weitere Fluidleitungen, die am Trägerelement angebracht sind, wobei jede von ihnen mindestens einen U-förmigen Schlauchabschnitt (45a, 48a, 50a, 51a) in Bezug auf das Trägerelement definiert, wobei jeder Schlauchabschnitt dafür vorgesehenist, mit einer entsprechenden Pumpe (3a, 3b, 3c, 3d, 3e) zusammenzuarbeiten, wobei
das Trägerelement (4) eine erste Zone (274), an welcher der Teil der Blutleitung (44) befestigt ist, und mindestens eine zweite Zone (275) gegenüber der ersten Zone aufweist, wobei die anderen Fluidleitungen (45, 48, 50, 51) alle in der zweiten Zone befestigt sind;
der U-förmige Schlauchabschnitt (44a) der Blutleitung (44) länger als die von den anderen Fluidleitungen definierten Schlauchabschnitte (45a, 48a, 50a, 51a) ist;
der U-förmige Schlauchabschnitt (44a) der Blutleitung einen größeren Krümmungsradius als die von den anderen Fluidleitungen definierten Schlauchabschnitte aufweist;
der Kreislauf zur Fluidverteilung (15) mindestens eine Ablassleitung (45) für ein Abfallfluid umfasst, wobei die Ablassleitung (45) für ein Abfallfluid am Trägerelement (4) angebracht ist und mindestens einen Schlauchabschnitt definiert, der als ein U (45a) am Trägerelement angeordnet ist, wobei der U-förmige Schlauchabschnitt (45a) dafür vorgesehenist, mit einer entsprechenden Pumpe (3b) zusammenzuarbeiten;
der Kreislauf zur Fluidverteilung (45) mindestens eine Aufnahmeleitung (48) für frische Dialyseflüssigkeit umfasst, wobei die Aufnahmeleitung (48) für frische Dialyseflüssigkeit am Trägerelement (4) befestigt ist und mindestens einen Schlauchabschnitt definiert, der als ein U (48a) am Trägerelement angeordnet ist, wobei der Schlauchabschnitt (48a) der Flüssigkeitsaufnahmeleitung dafür vorgesehenist, bei Verwendung mit einer entsprechenden Pumpe (3c) zusammenzuarbeiten;
das Modul ferner mindestens eine Fluidinfusionsleitung (51) umfasst, wobei die Fluidinfusionsleitung (51) an der Trägerleitung (4) befestigt ist und mindestens einen Schlauchabschnitt definiert, der als ein U (51a) am Trägerelement angeordnet ist, wobei die Fluidinfusionsleitung (51) dafür vorgesehenist, in Verwendung mit einer entsprechenden Pumpe (3d) zusammenzuarbeiten;
das Modul ferner mindestens eine Prä-Infusionshilfsleitung (50) umfasst, wobei die Prä-Infusionshilfsleitung (50) am Trägerelement (4) befestigt ist und mindestens einen Schlauchabschnitt definiert, der als ein U (50a) am Trägerelement angeordnet ist, wobei die Prä-Infusionshilfsleitung (50) dafür vorgesehenist, mit einer entsprechenden Pumpe (3e) zusammenzuarbeiten;
das Trägerelement (4) einen Hauptkörper (6) umfasst, der eine Stirnwand (25) und mindestens eine von der Stirnwand (25) vorspringende Umfangswand (32) aufweist, wobei die Stirnwand (25) und die Umfangswand (32) ein Gehäusefach (33) definieren;
die Stirnwand (25) mindestens erste Seiten (55, 56) und zweite Seiten (53, 54) umfasst, die im Wesentlichen parallel und einander zugewandt sind;
die Stirnwand von ersten gegenüberliegenden längeren Seiten (55, 56) mit einem im Wesentlichen geradlinigem Verlauf begrenzt wird, wobei jede zwei gekrümmte Teile (55a, 55b, 56a, 56b) aufweist, deren Kavität ihrer entsprechenden Gegenseite zugewandt ist;
der Schlauchabschnitt (48a) der Aufnahmeleitung (48) für frische Dialyseflüssigkeit an einer der ersten längeren Seiten (55, 56) befestigt ist;
der Schlauchabschnitt (45a) der Ablassleitung (45) für ein Abfallfluid an einer der ersten längeren Seiten (55, 56) befestigt ist;
der Schlauchabschnitt (51 a) der Fluidinfusionsleitung (51) an einer der ersten längeren Seiten (55, 56) befestigt ist;
der Schlauchabschnitt (50a) der Prä-Infusionshilfsleitung (50) an einer der ersten längeren Seiten (55, 56) befestigt ist;
sich die U-förmigen Schlauchabschnitte (44a, 45a, 48a, 50a, 51 a) in Bezug auf die Umfangswand (32) des Trägerelements (4) innen erstrecken.

2. Modul nach Anspruch 1, **dadurch gekennzeichnet, dass** die zweite Zone (275) mindestens zwei nebeneinander platzierte Teilhälften (275a, 275b) umfasst, wobei mindestens der Schlauchabschnitt der Ablassleitung (45) an der zweiten Teilhälfte (275b) befestigt ist.

3. Modul nach Anspruch 1 oder 2, **dadurch gekennzeichnet, dass** die zweite Zone (275) mindestens zwei nebeneinander platzierte Teilhälften (275a, 275b) umfasst, wobei mindestens der Schlauchabschnitt (48a) der Aufnahmeleitung (48) für frische Flüssigkeiten an der ersten Teilhälfte (275a) befestigt ist.

4. Modul nach einem der vorangehenden Ansprüche, **dadurch gekennzeichnet, dass** die zweite Zone (275) mindestens zwei nebeneinander platzierte Teilhälften (275a, 275b) umfasst, wobei mindestens der Schlauchabschnitt (51a) der Infusionsleitung (51) an der ersten Teilhälfte (275a) befestigt ist.

5. Modul nach einem der vorangehenden Ansprüche, **dadurch gekennzeichnet, dass** die zweite Zone (275) mindestens zwei nebeneinander platzierte Teilhälften (275a, 275b) umfasst, wobei mindestens der Schlauchabschnitt (50a) der Prä-Infusionshilfsleitung (50) an der zweiten Teilhälfte (275b) befestigt ist.

6. Modul nach einem der vorangehenden Ansprüche, **dadurch gekennzeichnet, dass** die Stirnwand von zweiten gegenüberliegenden kürzeren Seiten (53, 54) mit einem im Wesentlichen geradlinigen Verlauf begrenzt wird.

7. Modul nach Anspruch 6, **dadurch gekennzeichnet, dass** mindestens eine der zweiten Seiten (53) einen gekrümmten Teil (53a) aufweist, der zwischen zwei geradlinigen Abschnitten (53b, 53c) platziert ist, wobei die Kavität des gekrümmten Teils der Gegenseite zugewandt ist.

8. Modul nach Anspruch 7, **dadurch gekennzeichnet, dass** die erste Zone (274) zum Teil von mindestens einer der zweiten Seiten (53), die den gekrümmten Teil (53a) aufweisen, und durch einen Teil der ersten gegenüberliegenden längeren Seiten (55, 56) neben der zweiten Seite (54), die den gekrümmten Teil aufweist, begrenzt wird.

9. Modul nach Anspruch 7, **dadurch gekennzeichnet, dass** die zweite Zone (275) zum Teil von einer der zweiten Seiten (54) ohne den gekrümmten Teil und durch einen Teil der ersten gegenüberliegenden längeren Seiten (55, 56) neben der zweiten Seite (54) ohne den gekrümmten Teil begrenzt wird.

10. Modul nach einem der vorangehenden Ansprüche, **dadurch gekennzeichnet, dass** die erste Zone (274) des Trägerelements (4) unter der zweiten Zone (275) des Trägerelements platziert ist, wenn das Modul mit einem Gerät zur extrakorporalen Blutbehandlung (2) verbunden ist.

11. Modul nach einem der Ansprüche 2 bis 5, **dadurch gekennzeichnet, dass** die erste und zweite Teilhälfte (275a, 275b) der zweiten Zone (275) des Trägerelements (4) nebeneinander platziert sind, wenn das Modul (1) mit einem Gerät zur extrakorporalen Blutbehandlung (2) verbunden ist.

12. Modul nach Anspruch 7, **dadurch gekennzeichnet, dass** der Schlauchabschnitt (44a) der Blutleitung an der zweiten Seite (53), die den gekrümmten Teil (53a) aufweist, befestigt ist.

13. Modul nach Anspruch 7, **dadurch gekennzeichnet, dass** eine der zweiten Seiten (54) ohne den gekrümmten Teil keinen direkt daran befestigten Schlauchabschnitt aufweist.

14. Modul nach einem der vorangehenden Ansprüche, **dadurch gekennzeichnet, dass** ein Abschnitt eines jeden U-förmigen Schlauchteils (44a, 45a, 48a, 50a, 51a) kleiner oder gleich (πR + 2R) ist, wobei R der entsprechende Krümmungsradius des Schlauchabschnitts ist.

15. Modul nach einem der vorangehenden Ansprüche, **dadurch gekennzeichnet, dass** es ferner eine Trägerstruktur (64) umfasst, die mit dem Hauptkörper (6) verbunden ist und seitlich von diesem platziert ist.

16. Modul nach Anspruch 15, **dadurch gekennzeichnet, dass** die Trägerstruktur (64) mit dem Hauptkörper (6) an einer der längeren Seiten (55, 56) im Eingriff steht.

17. Modul nach Anspruch 16, **dadurch gekennzeichnet, dass** die Trägerstruktur (64) mit dem Hauptkörper (6) an den gekrümmten Teilen (55a, 55b, 56a, 56b) an einer der ersten längeren Seiten (55, 56) im Eingriff steht.

18. Modul nach einem der vorangehenden Ansprüche, **dadurch gekennzeichnet, dass** es ferner mindestens eine Einheit zur Blutbehandlung (5) umfasst, die mit dem Trägerelement (4) im Eingriff steht.

19. Modul nach einem der vorangehenden Ansprüche, **dadurch gekennzeichnet, dass** das Trägerelement (4) mindestens ein erstes und mindestens ein zweites Anschlussstück (7, 8) umfasst, die mit dem Hauptkörper (6) verbunden und voneinander beabstandet sind, wobei das erste und das zweite Anschlussstück (7, 8) dafür vorgesehen sind, durch Eingriff entsprechende Gegenanschlussstücke (9, 10) einer Einheit zur Blutbehandlung aufzunehmen, die auf dem Trägerelement zu montieren ist.

20. Modul nach den Ansprüchen 18 und 19, **dadurch gekennzeichnet, dass** die Behandlungseinheit (5) Folgendes umfasst:
- einen Gehäusekörper (40);
- mindestens eine im Gehäusekörper arbeitende semipermeable Membran (41), die eine erste und eine zweite Kammer definiert;
- ein erstes und ein zweites Gegenanschlussstück (9, 10), die mit dem Gehäusekörper (40) verbunden und an entsprechenden mit dem Hauptkörper (6) verbundenen Anschlussstücken (7, 8) befestigt sind, wobei mindestens eines des ersten und zweiten Gegenanschlussstücks (9, 10) mit der zweiten Kammer der Behandlungseinheit und mit entsprechenden ersten Endteilen der Anschlussstücke in Fluidverbindung gesetzt ist;
- mindestens eine in die erste Kammer führende Zugangsöffnung (42); und
- mindestens eine aus der ersten Kammer führende Ausgangsöffnung (43).

21. Modul nach den Ansprüchen 15 und 19, **dadurch gekennzeichnet, dass** die Anschlussstücke (7, 8) und die Trägerstruktur (64) seitlich vom Hauptkörper (6) an einer der längeren Seiten (55, 58) positioniert sind.

22. Modul nach einem der Ansprüche 2 bis 5, **dadurch gekennzeichnet, dass** die erste und zweite Teilhälfte (275a, 275b) spiegelsymmetrisch zu einer Längsachse des Hauptkörpers (6) sind.

23. Gerät zur extrakorporalen Blutbehandlung, das dazu bestimmt ist, ein integriertes Modul nach einem der vorangehenden Ansprüche aufzunehmen, wobei das Gerät einen Körper (100), aufweisend eine Oberfläche (101), einen Vorsprung zur Führung und Positionierung (102), der von der Oberfläche (101) hervorragt und genau gegensätzlich zu einer Umfangswand (32) eines Trägerelements geformt ist, mit dem er verkoppelt werden muss, eine bestimmte Anzahl von Pumpen (3a, 3b, 3c, 3d, 3e), die von der Oberfläche (101) hervorragen und dafür vorgesehen sind, mit einem geeigneten Kreislauf zur Fluidverteilung (15) des mit dem Gerät zu verbindenden integrierten Moduls zusammenzuarbeiten, umfasst, wobei mindestens ein Teil einer Seitenfläche der Pumpen (3a, 3b, 3c, 3d, 3e) gegensätzlich zur Umfangswand (32) des Trägerelements geformt ist, wobei die hervorragenden Pumpen und der Vorsprung zur Führung und Positionierung (102) gemeinsam Aufnahmen (104a, 104b, 104c, 104d und 104e) definieren, die im Wesentlichen eine Halbkreis- oder U-Form annehmen und dafür vorgesehensind, die entsprechenden U-förmigen Schlauchabschnitte (44a, 45a, 48a, 50a) des Kreislaufs zur Fluidverteilung (15) des integrierten Moduls aufzunehmen, wobei
mindestens eine der Pumpen eine Blutpumpe (3a) ist, die dafür vorgesehenist, mit einer entsprechenden Blutleitung (44) des Verteilungskreislaufs (15) zusammenzuarbeiten,
mindestens eine der Pumpen eine Zuführpumpe (3c) ist, die dafür vorgesehenist, mit einer entsprechenden Zuführleitung (48) für frische Dialyseflüssigkeit des Verteilungskreislaufs (15) zusammenzuarbeiten,
mindestens eine der Pumpen eine Saugpumpe (3b) ist, die dafür vorgesehenist, mit einer entsprechenden Ablassleitung (45) des Verteilungskreislaufs (15) zusammenzuarbeiten,
mindestens eine der Pumpen eine Infusionspumpe (3d) ist, die dafür vorgesehenist, mit einer entsprechenden Infusionsleitung (51) des Verteilungskreislaufs (15) zusammenzuarbeiten,
mindestens eine der Pumpen eine Prä-Infusionshilfspumpe (3e) ist, die dafür vorgesehenist, mit einer entsprechenden Prä-Infusionshilfsleitung (50) des Verteilungskreislaufs (15) zusammenzuarbeiten, wobei der Gerätekörper (100) auf seiner Oberfläche (101) eine erste Zone (174), die die Blutpumpe (3a) aufweist, und mindestens eine zweite Zone (175), die der ersten Zone gegenüberliegt und die anderen Pumpen (3b, 3c, 3d, 3e) aufweist, definiert.

24. Gerät nach Anspruch 23, **dadurch gekennzeichnet, dass** die zweite Zone (175) mindestens zwei nebeneinander platzierte Teilhälften (175a, 175b) umfasst, wobei die Zuführpumpe (3c) in der ersten Teilhälfte (175a) platziert ist.

25. Gerät nach einem der Ansprüche 23 und 24, **dadurch gekennzeichnet, dass** die erste Zone (174) des Gerätekörpers (100) unter Betriebsbedingungen unterhalb der zweiten Zone (175) des Körpers platziert ist.

26. Gerät nach Anspruch 24, **dadurch gekennzeichnet, dass** die erste und zweite Teilhälfte (175a, 175b) der zweiten Zone (175) des Gerätekörpers (100) unter Betriebsbedingungen nebeneinander platziert sind.

27. Gerät nach Anspruch 24, **dadurch gekennzeichnet, dass** die erste und zweite Teilhälfte (175a, 175b) spiegelsymmetrisch sind.

28. Gerät nach einem der Ansprüche 23 bis 27, **dadurch gekennzeichnet, dass** die zweite Zone (175) mindestens zwei nebeneinander platzierte Teilhälften (175a, 175b) umfasst, wobei die Saugpumpe (3b) in der zweiten Teilhälfte (175b) platziert ist.

29. Gerät nach einem der Ansprüche 23 bis 28, **dadurch gekennzeichnet, dass** die zweite Zone (175) mindestens zwei nebeneinander platzierte Teilhälften (175a, 175b) umfasst, wobei die Infusionspumpe (3d) in der ersten Teilhälfte (175a) platziert ist.

30. Gerät nach einem der Ansprüche 23 bis 29, **dadurch gekennzeichnet, dass** die zweite Zone (175) mindestens zwei nebeneinander platzierte Teilhälften (175a, 175b) umfasst, wobei die Prä-Infusionshilfspumpe (3e) in der zweiten Teilhälfte (175b) platziert ist.

## Revendications

1. Module intégré de traitement du liquide, comprenant :
- un élément de support (4) ;
- une circuiterie de distribution du liquide (15) associée à l'élément de support (4) et comprenant :
au moins une ligne à sang (44), au moins une portion de ladite ligne à sang étant attachée à l'élément de support (4) et définissant au moins un morceau de tube en forme de U (44a) par rapport audit élément de support, ledit morceau de tube (44a) étant conçu pour coopérer avec une pompe respective (3a) ;
d'autres lignes de liquides fixées à l'élément de support, définissant chacune au moins un morceau de tube en forme de U (45a, 48a, 50a, 51a), par rapport audit élément de support, chaque morceau de tube (44a) étant conçu pour coopérer avec une pompe respective (3a, 3b, 3c, 3d, 3e), dans lequel l'élément de support (4) présente une première zone (274) à laquelle est attachée la portion de la ligne à sang (44), et au moins une deuxième zone (275) opposée à ladite première zone, les autres lignes de liquides (45, 48, 50, 51) étant toutes attachées à ladite deuxième zone ;
le morceau de tube en forme de U (44a) de la ligne à sang (44) est plus long que les morceaux de tube (45a, 48a, 50a, 51a) définis par les autres lignes de liquides ;
le morceau de tube en forme de U (44a) de la ligne à sang (44) présente un rayon de courbure plus grand que les morceaux de tube définis par les autres lignes de liquides ;
la circuiterie de distribution du liquide (15) comprend au moins une ligne d'évacuation (45) pour un déchet de liquide, ladite ligne d'évacuation (45) pour un déchet de liquide étant fixée à l'élément de support (4) et définissant au moins un morceau de tube disposé en U (45a) par rapport audit élément de support, ledit morceau de tube en forme de U (45a) étant conçu pour coopérer avec une pompe respective (3b) ;
la circuiterie de distribution du liquide (15) comprend au moins une ligne d'aspiration (48) pour du liquide de dialyse frais, la ligne d'aspiration (48) pour du liquide de dialyse frais étant attachée à l'élément de support (4) et définissant au moins un morceau de tube disposé en U (48a) par rapport audit élément de support, ledit morceau de tube (48a) de la ligne d'aspiration du liquide étant conçue pour coopérer, lors de son utilisation, avec une pompe respective (3c) ;
le module comprend également au moins une ligne pour le liquide de perfusion (51), la ligne pour le liquide de perfusion (51) étant attachée à la ligne de support (4) et définissant au moins un morceau de tube disposé en U (51a) par rapport audit élément de support, ladite ligne pour le liquide de perfusion (51) étant conçue pour coopérer, lors de son utilisation, avec une pompe respective (3d) ;
le module comprend également au moins une ligne auxiliaire de pré-perfusion (50), la ligne auxiliaire de pré-perfusion (50) étant attachée à l'élément de support (4) et définissant au moins un morceau de tube disposé en U (50a) par rapport audit élément de support, ladite ligne auxiliaire de pré-perfusion (50) étant conçue pour coopérer avec une pompe respective (3e) ;
l'élément de support {4) comprend un corps principal (6) présentant une paroi antérieure (25) et au moins une paroi périphérique (32) faisant saillie en s'éloignant de ladite paroi antérieure (25), ladite paroi antérieure (25) et ladite paroi périphérique (32) définissant un compartiment de logement (33) ;
la paroi antérieure (25) comprend au moins des premiers côtés (55, 56) et des seconds côtés (53, 54) fondamentalement parallèles et orientés réciproquement ;
la paroi antérieure est délimitée par des premiers côtés opposés plus longs (55, 56) présentant un développement fondamentalement rectiligne, muni chacun de deux portions courbes (55a, 55b, 56a, 56b) dont la cavité est orientée vers son côté opposé respectif;
le morceau de tube (48a) de la ligne d'aspiration (48) pour le liquide de dialyse frais est attaché à l'un desdits premiers côtés plus longs (55, 56);
le morceau de tube (45a) de la ligne d'évacuation (45) pour un déchet de liquide est attaché à l'un desdits premiers côtés plus longs (55, 56);
le morceau de tube (51a) de la ligne pour le liquide de perfusion (51) est attaché à l'un desdits premiers côtés plus longs (55, 56) ;
le morceau de tube (50a) de la ligne auxiliaire de pré-perfusion (50) est attaché à l'un desdits premiers côtés plus longs (55, 56) ;
les morceaux de tube en forme de U (44a, 45a, 48a, 50a, 51a) se prolongent à l'intérieur par rapport à la paroi périphérique (32) de l'élément de support (4).

2. Module selon la revendication 1, **caractérisé en ce que** ladite deuxième zone (275) comprend au moins deux moitiés placées côte à côte (275a, 275b), au moins le morceau de tube de la ligne d'évacuation (45) étant attaché à la deuxième moitié (275b).

3. Module selon la revendication 1 ou 2, **caractérisé en ce que** ladite deuxième zone (275) comprend au moins deux moitiés placées côte à côte (275a, 275b), au moins le morceau de tube (48a) de la ligne d'aspiration (48) pour le liquide frais étant attachée à la première moitié (275a).

4. Module selon l'une quelconque des revendications précédentes, **caractérisé en ce que** ladite deuxième zone (275) comprend au moins deux moitiés placées côte à côte (275a, 275b), au moins le morceau de tube (51a) de la ligne de perfusion (51) étant attaché à la première moitié (275a).

5. Module selon l'une quelconque des revendications précédentes, **caractérisé en ce que** ladite deuxième zone (275) comprend au moins deux moitiés placées côte à côte (275a, 275b), au moins le morceau de tube (50a) de la ligne auxiliaire de pré-perfusion (50) étant attaché à la deuxième moitié (275b).

6. Module selon l'une quelconque des revendications précédentes, **caractérisé en ce que** la paroi antérieure est délimitée des deuxièmes côtés opposés plus courts (53, 54) présentant un développement fondamentalement rectiligne.

7. Module selon la revendication 6, **caractérisé en ce qu'**au moins un desdits seconds côtés (53) présente une portion courbe (53a) placée entre deux morceaux rectilignes (53b, 53c), la cavité de ladite portion courbe étant orientée vers le côté opposé.

8. Module selon la revendication 7, **caractérisé en ce que** la première zone (274) est partiellement délimitée par au moins un desdits seconds côtés (53) présentant la portion courbe (53a) et par une portion des premiers côtés opposés plus longs (55, 56) à côté dudit second côté (54) présentant la portion courbe.

9. Module selon la revendication 7, **caractérisé en ce que** la deuxième zone (275) est partiellement délimitée par au moins un desdits seconds côtés (54) sans la portion courbe et par une portion des premiers côtés opposés plus longs (55, 56) à côté dudit second côté (54) sans la portion courbe.

10. Module selon l'une quelconque des revendications précédentes, **caractérisé en ce que**, quand le module est associé à un appareil de traitement du sang extracorporel (2), la première zone (274) de l'élément de support (4) est placée en dessous de la deuxième zone (275) de l'élément de support.

11. Module selon l'une quelconque des revendications 2 à 5, **caractérisé en ce que**, quand le module (1) est associé à un appareil de traitement du sang extracorporel (2), la première et la deuxième moitiés (275a, 275b) de la deuxième zone (275) de l'élément de support (4) sont placées côte à côte.

12. Module selon la revendication 7, **caractérisé en ce que** le morceau de tube (44a) de la ligne à sang est attaché audit deuxième côté (53) présentant la portion courbe (53a).

13. Module selon la revendication 7, **caractérisé en ce qu'**aucun morceau de tube n'est directement attaché à l'un desdits seconds côtés (54) sans la portion courbe.

14. Module selon l'une quelconque des revendications précédentes, **caractérisé en ce qu'**un morceau de chaque portion de tube en forme de U (44a, 45a, 48a, 50a, 51a) est plus petit ou identique à (πR + 2R), où R est le rayon de courbure respectif du morceau de tube.

15. Module selon l'une quelconque des revendications précédentes, **caractérisé en ce qu'**il comprend également une structure de support (64) associée au corps principal (6) et placée latéralement par rapport à cette dernière.

16. Module selon la revendication 15, **caractérisé en ce que** ladite structure de support (64) est engagée sur le corps principal (6) de l'un desdits côtés plus longs (55, 56).

17. Module selon la revendication 16, **caractérisé en ce que** ladite structure de support (64) est engagée sur le corps principal (6) sur lesdites portions courbes (55a, 55b, 56a, 56b) de l'un desdits premiers côtés plus longs (55, 56).

18. Module selon l'une quelconque des revendications précédentes, **caractérisé en ce qu'**il comprend également au moins une unité de traitement du sang (5) engagée sur l'élément de support (4).

19. Module selon l'une quelconque des revendications précédentes, **caractérisé en ce que** ledit élément de support (4) comprend au moins un premier et au moins un deuxième connecteurs (7, 8) associés au corps principal (6) et espacés l'un de l'autre, ledit premier et ledit deuxième connecteurs (7, 8) étant conçus pour recevoir par engagement des connecteurs de comptage (9, 10) correspondants d'une unité de traitement du sang à monter sur l'élément de support.

20. Module selon les revendications 18 et 19, **caractérisé en ce que** l'unité de traitement (5) comprend :
- un corps de logement (40) ;
- au moins une membrane semi-perméable (41) fonctionnant à l'intérieur du corps de logement définissant une première et une deuxième chambre ;
- un premier et un deuxième connecteur de comptage (9, 10) associés au corps de logement (40) et attachés aux connecteurs respectifs (7,8) associés à un corps principal (6), au moins un desdits premier et deuxième connecteurs (9, 10) étant placé en communication de fluide avec la deuxième chambre de l'unité de traitement et avec des premières portions terminales respectives desdits connecteurs ;
- au moins un orifice d'accès (42) à ladite première chambre ; et
- au moins un orifice de sortie (43) de ladite première chambre.

21. Module selon les revendications 15 et 19, **caractérisé en ce que** les connecteurs (7, 8) et la structure de support (64) sont positionnés latéralement par rapport au corps principal (6) sur l'un desdits côtés plus longs (55, 58).

22. Module selon les revendications 2 à 5, **caractérisé en ce que** la première et la deuxième moitié (275a, 275b) sont symétriques de façon spéculaire par rapport à un axe longitudinal du corps principal (6).

23. Appareil pour le traitement extracorporel du sang destiné à recevoir un module intégré selon l'une quelconque des revendications précédentes, l'appareil comprenant un corps (100) présentant une surface (101), une saillie de guidage et de positionnement (102) dépassant de la surface (101) et ayant exactement une forme complémentaire à une paroi périphérique (32) d'un élément de support auquel il doit être accouplé, un nombre donné de pompes (3a, 3b, 3c, 3d, 3e) dépassant de la surface (101) et conçues pour coopérer avec une circuiterie de distribution du liquide appropriée (15) du module intégré à associer à l'appareil, au moins une partie de la surface d'un côté des pompes (3a, 3b, 3c, 3d, 3e) a une forme complémentaire à la paroi périphérique (32) de l'élément de support, les pompes dépassant et la saillie de guidage et de positionnement (102) définissent ensemble des sièges (104a, 104b, 104c, 104d et 104e) prenant une forme fondamentalement en demi-cercle ou en forme de U et conçues pour recevoir les morceaux de tube correspondants en forme de U (44a, 45a, 48a, 50a) de la circuiterie de distribution du liquide (15) du module intégré, dans lequel
au moins une desdites pompes étant une pompe à sang (3a) conçue pour coopérer avec une ligne à sang respective (44) de la circuiterie de distribution (15),
au moins une desdites pompes est une pompe d'alimentation (3c) conçue pour coopérer avec une ligne d'alimentation respective (48) pour le liquide de dialyse frais de la circuiterie de distribution (15),
au moins une desdites pompes est une pompe d'aspiration (3b) conçue pour coopérer avec une ligne d'évacuation respective (45) de la circuiterie de distribution (15),
au moins une desdites pompes est une pompe de perfusion (3d) conçue pour coopérer avec une ligne de perfusion respective (51) de la circuiterie de distribution (15),
au moins une desdites pompes est une pompe auxiliaire de pré-perfusion (3e) conçue pour coopérer avec une ligne auxiliaire de pré-perfusion (50) de la circuiterie de distribution (15), le corps de l'appareil (100) définissant sur sa surface (101) une première zone (174) présentant ladite pompe à sang (3a) et au moins une deuxième zone (175) opposée à ladite première zone et présentant les autres pompes (3b, 3c, 3d, 3e).

24. Appareil selon la revendication 23, **caractérisé en ce que** ladite deuxième zone (175) comprend au moins deux moitiés placées côte à côte (175a, 175b), la pompe d'alimentation (3c) étant placée dans ladite première moitié (175a).

25. Appareil selon l'une quelconque des revendications 23 et 24, **caractérisé en ce que**, dans des conditions opérationnelles, la première zone (174) du corps de l'appareil (100) est placée en dessous de la deuxième zone (175) dudit corps.

26. Appareil selon la revendication 24, **caractérisé en ce que**, dans des conditions opérationnelles, la première et la deuxième moitié (175a, 175b) de la deuxième zone (175) du corps de l'appareil (100) sont placées côte à côte.

27. Appareil selon la revendication 24, **caractérisé en ce que** lesdites première et deuxième moitiés (175a, 175b) sont symétriques de façon spéculaire.

28. Appareil selon l'une quelconque des revendications 23 à 27, **caractérisé en ce que** ladite deuxième zone (175) comprend au moins deux moitiés placées côte à côte (175a, 175b), la pompe d'aspiration (3b) étant placée dans ladite deuxième moitié (175b).

29. Appareil selon l'une quelconque des revendications 23 à 28, **caractérisé en ce que** ladite deuxième zone (175) comprend au moins deux moitiés placées côte à côte (175a, 175b), la pompe de perfusion (3d) étant placée dans ladite première moitié (175a).

30. Appareil selon l'une quelconque des revendications 23 à 29, **caractérisé en ce que** la dite deuxième zone (175) comprend au moins deux moitiés placées côte à côte (175a, 175b), la pompe auxiliaire de pré-perfusion (3e) étant placée dans ladite deuxième moitié (175b).
